# EUROPEAN PATENT SPECIFICATION

(11) **EP 2 291 543 B1**
(45) Date of publication and mention of the grant of the patent: **15.04.2015**
(21) Application number: 09747650.1
(22) Date of filing: 15.05.2009
(51) Int. Cl.: C12Q 1/68

(54) **GENETIC MARKERS FOR WEIGHT MANAGEMENT AND METHODS OF USE THEREOF**
GENETISCHE MARKER ZUR GEWICHTSKONTROLLE UND VERWENDUNGSVERFAHREN DAFÜR
MARQUEURS GÉNÉTIQUES COMME OUTIL DE GESTION DU POIDS ET PROCÉDÉS D'UTILISATION DE CEUX-CI

(30) Priority: 16.05.2008 US 53888 P
(43) Date of publication of application: 09.03.2011
(73) Proprietor: Interleukin Genetics, Inc., Waltham, MA 02452 (US); Access Business Group International LLC, Ada, Michigan 49355 (US)
(72) Inventor: DRAPER, Colleen, Stoneham, MA 02180 (US); WILKINS, Leon, N. Andover, MA 01845 (US); BRETON, Gary, Marlborough, MA 01752 (US); PERUSSE, Louis, Quebec, GIV0A6 (CA); DEBUSK, Ruth, Tallahassee, FL 32303-5304 (US); KREMPIN, David, - (US); RAMAKRISHNAN, Shyam, Whittier, CA 90603 (US)
(74) Representative: Mintz Levin Cohn Ferris Glovsky and Popeo LLP
(86) International application number: PCT/US2009/044076
(87) International publication number: WO 2009/140569

(56) References cited:
- WO-A-2005/079325
- SORENSEN THORKILD I A ET AL: "Genetic polymorphisms and weight loss in obesity: a randomised trial of hypo-energetic high- versus low-fat diets" PLOS CLINICAL TRIALS, PUBLIC LIBRARY OF SCIENCE, US, vol. 1, no. 2, 1 June 2006 (2006-06-01), pages 1-14, XP002467235 ISSN: 1555-5887
- MARTI AMELIA ET AL: "Interaction between genes and lifestyle factors on obesity" PROCEEDINGS OF THE NUTRITION SOCIETY, vol. 67, February 2008 (2008-02), pages 1-8, XP002554347 ISSN: 0029-6651
- DE LUIS D A ET AL: "Influence of ALA54THR polymorphism of fatty acid binding protein 2 on lifestyle modification response in obese subjects" ANNALS OF NUTRITION & METABOLISM, vol. 50, no. 4, 2006, pages 354-360, XP008114557 ISSN: 0250-6807 cited in the application
- MEMISOGLU ASLI ET AL: "Interaction between a peroxisome proliferator-activated receptor gamma gene polymorphism and dietary fat intake in relation to body mass." HUMAN MOLECULAR GENETICS, vol. 12, no. 22, 15 November 2003 (2003-11-15), pages 2923-2929, XP002554348 ISSN: 0964-6906 cited in the application
- MARTI A ET AL: "TRP64ARG polymorphism of the beta 3-adrenergic receptor gene and obesity risk: effect modification by a sedentary lifestyle." DIABETES, OBESITY & METABOLISM NOV 2002, vol. 4, no. 6, November 2002 (2002-11), pages 428-430, XP002554349 ISSN: 1462-8902
- UKKOLA O ET AL: "Beta-2 adrenergic receptor variants are associated with subcutaneous fat accumulation in response to long-term overfeeding." INTERNATIONAL JOURNAL OF OBESITY AND RELATED METABOLIC DISORDERS : JOURNAL OF THE INTERNATIONAL ASSOCIATION FOR THE STUDY OF OBESITY NOV 2001, vol. 25, no. 11, November 2001 (2001-11), pages 1604-1608, XP002554350 ISSN: 0307-0565 cited in the application

## Description

### FIELD OF THE INVENTION

This application relates to methods of determining a subject's metabolic genotype and methods for selecting an appropriate therapeutic/dietary regimen or lifestyle recommendation based on the subject's metabolic profile and susceptibility to adverse weight management issues.

### BACKGROUND

According to a report published in 1998 by the World Health Organization (WHO), obesity has reached epidemic proportions worldwide: about 1.7 billion people worldwide are overweight and 300 million of them are obese. In the U.S. approximately 127 million adults are overweight and 69 million are obese. Obese subjects are at increases risk of developing one or more serious medical conditions including diabetes, heart disease, high blood pressure and high blood cholesterol. The prevalence of obesity has more than doubled in the past 25 years and now reaches 31% among U.S. adults aged 20 years and older. Higher rates of obesity are seen among African-Americans and Hispanic Americans, especially among women (30% to 50%).

The increase in the prevalence of obesity observed worldwide in the past decades has occurred in a changing environment characterized by a progressive reduction of physical activity level and the abundance of highly palatable foods. The WHO Report identified these changes as the two principal modifiable characteristics of modern lifestyle promoting the development of obesity. However, despite the fact that people are exposed to the same environment, not everyone is becoming obese, suggesting a role for a subject's genetic profile in the development of weight management issues. That is, genetics determines a subject's susceptibility to become obese when exposed to a unfavorable environment as well as the way he/she can respond to diet and exercise.

Sorensen et al, 2006, (PLOS Clinical Trials Public Library of Science, US 1(2): p1-14), reports a method for investigating the effect of a panel of 42 SNPs in 26 candidate genes in diet-induced weight loss. Therefore, analysis of a relatively large set of SNPs is required.

Marti et al, 2008, (Proceedings of the Nutrition Society, 67: p1-8) discusses obesity risk depending on (1) genetic variants (SNPs) and (2) exposure to environmental risks. 22 genes thought to be involved in obesity are discussed, including PPARy2 (Pro12Ala), β-adrenoceptor 2 (Gln27Glu) and uncoupling proteins 1, 2, 3.

WO2005/079325 relates to a method for assessing susceptibility of a subject to a genetically related disease, including determining the presence or absence of risk factors correlated with the disease, assigning a risk score to each of the risk factors present and combining risk scores to calculate an overall susceptibility score. In relation to obesity, a number of genes affecting energy balance are described.

De Luis et al, 2006, (Annals of Nutrition and Metabolism 50 (4): p 354-360) discloses the influence of the FABP2 Thr54 polymorphism (rs1799883) on response to lifestyle modification, including in obese patients. A single polymorphic locus is examined and related to a particular hypocaloric diet.

Memisoglu et al, 2003, (Human Molecular Genetics, 12(22): p2923-9) discusses the interaction between a PPARG polymorphism and dietary fat intake in relation to body mass.

Marti et al, 2002, (Diabetes, Obesity and Metabolism 4/6: p428-430) reports results suggesting that the TRP64/ARG polymorphism of the ADRB3 gene seems to be a risk factor for obesity that is dependent on a sedentary lifestyle.

Ukkola et al, 2001, (International Journal of Obesity and Related Metabolic Disorders: Journal of the International Association for the Study of Obesity, 25(11): p1604-8) reports that Beta-2 adrenergic receptor variants are associated with subcutaneous fat accumulation in response to long-term overfeeding.

Accordingly, there is a need for a means for establishing a personalized weight loss program that considers a person's genetic susceptibility to obesity in order to improve weight loss and weight maintenance outcomes relative to a similar program not taking into account genetic information. There is a need for a means for linking a subject's metabolic genotype to response to diet and/or exercise.

The description herein of disadvantages and problems associated with known methods is in no way intended to limit the scope of the embodiments described in this document to their exclusion.

### SUMMARY OF THE INVENTION

The present invention provides for methods and kits for determining a subject's metabolic genotype and selecting an appropriate therapeutic/dietary regimen or lifestyle recommendation for the subject. According to some embodiments, methods are provided for determining a subject's metabolic genotype, classifying the subject into one or more of a series of nutritional and exercise categories to which the subject is likely to be responsive, and communicating to the subject an appropriate therapeutic/dietary regimen or lifestyle recommendation for the subject. In this manner, a personalized weight-loss program may be chosen based on a subject's metabolic genotype. Such a personalized weight-loss program will have obvious benefits (e.g., yield better results in terms of weight loss and weight maintenance) over traditional weight-loss programs that do not take into account genetic information.

The invention provides a method for selecting an appropriate therapeutic dietary regimen recommendation for a subject comprising determining the subject's genotype with respect to the FABP2 (rs1799883; G/A); PPARG (rs1801282; C/G); and ADRB2 (rs1042714; C/G) polymorphic loci and at least one of the ADRB3 (rs 4994; C/T) locus and the ADRB2 (rs 1042713; A/G) locus; wherein the subject's genotype with respect to said loci provides information about the subject's increased susceptibility to adverse weight management issues and allows selection of a therapeutic/dietary regimen that is suitable for the subject, and wherein carrying the ADRB2 Glu27 allele, PPARG 12Pro/Pro and FABP2 54Thr/* qualifies the subject as responsive to fat restriction. Optionally, the method comprises determining the subject's genotype with respect to both the ADRB2 (rs4994; C/T) locus and the ADRB2 (rs1042713; A/G) locus.

A method is disclosed for selecting an appropriate therapeutic/dietary regimen or lifestyle recommendation for a subject comprising: determining a subject's genotype with respect to any two, any three, or any four of the polymorphic loci selected from the FABP2 (rs1799883; G/A) locus, PPARG (rs1801282; C/G) locus, ADRB3 (rs4994; C/T) locus, ADRB2 (rs1042713; A/G) locus, or ADRB2 (rs1042714; C/G) locus, wherein the subject's genotype with respect to said loci provides information about the subject's increased susceptibility to adverse weight management issues, and allows the selection of a therapeutic/dietary regimen or lifestyle recommendation that is suitable to the subject's susceptibility to adverse weight management issues.

Methods are disclosed for selecting an appropriate therapeutic/dietary regimen or lifestyle recommendation for a subject comprising: a) determining the subject's genotype with respect to the FABP2 (rs1799883; G/A) locus, PPARG (rs1801282; C/G) locus, ADRB3 (rs4994; C/T) locus, ADRB2 (rs1042713; A/G) locus, or ADRB2 (rs1042714; C/G) locus, wherein the subject's genotype with respect to said loci provides information about the subject's increased susceptibility to adverse weight management issues, and allows the selection of a therapeutic/dietary regimen or lifestyle recommendation that is suitable to the subject's susceptibility to adverse weight management issues.

Methods are disclosed for selecting an appropriate therapeutic/dietary regimen or lifestyle recommendation for a subject comprising: a) determining the subject's genotype with respect to any two, any three, or any four of the polymorphic loci selected from the group consisting of the FABP2 (rs1799883; G/A) locus, PPARG (rs1801282; C/G) locus, ADRB3 (rs4994; C/T) locus, ADRB2 (rs1042713; A/G) locus, or ADRB2 (rs1042714; C/G) locus and, b) classifying the subject's genotype into a nutrition responsiveness category and/or an exercise responsiveness category. Once a subject's genotype is classified or categorized into a nutrition responsiveness category and/or an exercise responsiveness category, a therapeutic/dietary regimen or lifestyle recommendation may be provided to the subject including, but not limited to, selecting an appropriate diet and activity level for which the subject is likely to be most responsive.

Methods are disclosed for selecting an appropriate therapeutic/dietary regimen or lifestyle recommendation for a subject comprising: a) determining the subject's genotype with respect to the FABP2 (rs1799883; G/A) locus, PPARG (rs1801282; C/G) locus, ADRB3 (rs4994; C/T) locus, ADRB2 (rs1042713; A/G) locus, or ADRB2 (rs1042714; C/G) locus and, b) classifying the subject's genotype into a nutrition responsiveness category and/or an exercise responsiveness category.

Methods are disclosed for selecting an appropriate therapeutic/dietary regimen or lifestyle recommendation for a subject comprising: (a) detecting an allelic pattern of at least two, at least three, at least four, at least five, at least six, at least seven, or at least eight alleles selected from the following: FABP2 SNP rs1799883, allele I (genotype: G; amino acid: Ala); FABP2 SNP rs1799883, allele 2 (genotype: A; amino acid: Thr); PPARG SNP rs1801282, allele 1 (genotype: C; amino acid: Pro); PPARG SNP rs1801282, allele 2 (genotype: G; amino acid: Ala); ADRB3 SNP rs4994, allele 1 (genotype: T; amino acid: Trp); ADRB3 SNP rs4994, allele 2 (genotype: C; amino acid: Arg); ADRB2 SNP rs1042713, allele 1 (genotype: G; amino acid: Gly); ADRB2 SNP rs1042713, allele 2 (genotype: A; amino acid: Arg); ADRB2 SNP rs1042714, allele 1 (genotype: C; amino acid: Gln); and ADRB2 SNP rs1042714, allele 2 (genotype: G; amino acid: Glu) locus, wherein the presence of allelic pattern is predictive of the subject's response to diet and/or exercise and (b) selecting a therapeutic/dietary regimen or lifestyle recommendation that is suitable for the subject's predicted response to diet and/or exercise.

Methods are disclosed for identifying a subject's metabolic genotype comprising: identifying the subject's genotype with respect to at least two, at least three, or at least four of the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

Methods are disclosed for identifying a subject's metabolic genotype comprising: identifying the subject's genotype with respect to the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

In addition we disclose kits which include a means for determining a subject's genotype with respect the subject's genotype with respect to the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus. The kit may also contain a sample collection means. The kit may also contain a control sample either positive or negative or a standard and/or an algorithmic device for assessing the results and additional reagents and components.

Kits may be in the form of a DNA test that will be used to provide diet and exercise recommendation based on a subject's genotype with respect to the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus. Information provided by a subject's genotype can help health professionals to develop personalized dietary and exercise interventions that will improve the prevention and treatment of obesity.

Other embodiments and advantages of the invention are set forth in the following detailed description and claims.

### DETAILED DESCRIPTION OF THE PREFERRED EMBODIMENTS

The methods of the present invention rely at least in part upon the finding that there is an association between the patterns of alleles of certain metabolic genes and the responsiveness of a subject to particular diet and exercise regime. That is, there is an association between the patterns of alleles of metabolic genes and weight management-related clinical outcomes and phenotypes. Certain genes impact various pathways that influence body weight, and have been associated with elevated risk for obesity and for their ability to differentiate subject's response to weight management interventions by genotype. For the purposes of this invention, such genes will be referred to as "metabolic genes" or "weight management genes". These genes include, but are not limited to, fatty acid binding protein 2 (FABP2); peroxisome proliferator-activated receptor-gamma (PPARG); beta-2 adrenergic receptor (ADRB2); and beta-3 adrenergic receptor (ADRB3).

The present invention relates to Weight Management Tests to determine a subject's "metabolic genotype", which involves determining a subject's genotype for one or more (*e.g.,* 2, 3, 4, etc) metabolic genes. The results of such metabolic genotyping may be used to predict a subject's responsiveness to relative amounts of macronutrients and calorie restriction in the diet, with or without exercise, for weight loss. Identifying a subject's genotype may be used to pairing the subject with a therapeutic, or nutrition, or lifestyle alteration, or a combination thereof to devise a strategy to achieve and/or sustain weight loss. Thus, according to some embodiments, polymorphism genotyping results (for single polymorphisms or combinations) may be used to determine 1) genetic influence on weight management intervention/outcomes and 2) responsiveness to macronutrients and energy restriction in the diet, with or without exercise, for weight loss.

Collectively, determination of a subject's genotype for one or more metabolic genes allows interpretations that provide actionable information for selecting an appropriate therapeutic/dietary regimen or lifestyle recommendation for a subject. A subject's metabolic genotype is determined from a Weight Management Test designed to detect a subject's genetic polymorphism pattern with respect to one or more metabolic gene. By identifying relevant gene polymorphisms and genotype pattern results, the test can assess risk for likely weight management outcomes and provide the subject with guidance on the choice of nutrition and lifestyle interventions that match their personal genetic makeup.

### METABOLIC GENES

Metabolic genes include, but are not limited to, fatty acid binding protein 2 (FABP2); peroxisome proliferator-activated receptor-gamma (PPARG); beta-2 adrenergic receptor (ADRB2); and beta-3 adrenergic receptor (ADRB3). A subject's genetic polymorphism pattern with respect to one or more of these genes reveals a subject's metabolic genotype. More preferably, a subject's metabolic genotype may be determined by identifying that subject's genetic polymorphism pattern with respect to one or more (*i.e.,* 2, 3, 4, or 5) of the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

### FABP2 rs1799883 (Ala54Thr; G/A) polymorphism

The FABP2 gene encodes the intestinal form of fatty acid binding protein, a family of proteins that regulates lipid transport and metabolism. FABP2 protein is found in small intestine epithelial cells where it controls fat absorption. In vitro, the Thr54 form of the protein shows a 2-fold greater binding affinity for long-chain fatty acids (Baier et al., J Clin Invest 95: 1281-1287, 1995) and was shown to be associated with enhanced fat absorption in the intestine (Levy et al., J Biol Chem 276: 39679-39684, 2001). The Thr54 variant thus increases absorption and/or processing of dietary fatty acids by the intestine and thereby increases fat oxidation. According to the most recent obesity gene map, a total of 5 studies showed evidence of association between FABP2 gene and obesity; four of them involved the Ala54Thr polymorphism. The 54Thr variant has been associated with elevated BMI and body fat (Hegele et al., Clin Endocrinol Metab 81: 4334-4337, 1996), increased abdominal fat in Japanese men (Yamada et al., Diabetologia 40: 706-710, 1997) and obesity as well as higher leptin levels among women (Albala et al., Obes Res 12: 340-345, 2004).

Multiple studies showed that the Ala54Thr polymorphism affects the response to changes of dietary fat in test meals. Non-esterified fatty acids (NEFA) were 20% higher 7 hours after a high-fat meal in 54Thr/Thr homozygote subjects compared with 54Ala/Ala homozygotes (Pratley et al., JLipid Res 41: 2002-2008, 2000). After fat ingestion, the 54Thr allele was also found to be associated with increased levels of postprandial triglycerides (Agren et al., Arterioscler Thromb Vasc Biol 18: 1606-1610, 1998) and 14-18 carbon chain fatty acids (Agren et al., Am J Clin Nutr 73: 31-35, 2001). The postprandrial metabolic profiles after test meals enriched with trans-fatty acids relative to a similar meal enriched with cis-fatty acids showed that subjects with at least one copy of the Thr54 allele exhibited a greater increase in postprandial glucose levels and lipogenesis compared to those homozygous for the Ala54 allele (Lefevre et al., Metabolism 54: 1652-1658, 2005). A group of obese, non-diabetic patients analyzed before and 3 months after a lifestyle modification program, consisting of hypocaloric diet (1,520 kcal/day) and aerobic exercise three times per week, (de Luis DA et al., Ann Nutr Metab 50: 354-360, 2006) showed that carriers of the 54Thr allele (compared to the wild-type 54Ala/Ala homozygotes) failed to have a significant reduction in fat mass, LDL-cholesterol levels, and leptin levels. Other studies have demonstrated an association between FABP2 genotype and dietary fat intake, with moderate carbohydrate intake (Marin et al., Am J Clin Nutr 82: 196-200, 2005; Takakura et al., Diabetes Research and Clinical Practice 67: 36-42, 2005).

### PPARG rs1801282 (C/G; Pro12Ala) polymorphism

The peroxisome proliferator-activated receptors (PPARs) are members of the nuclear hormone receptor subfamily of transcription factors. PPAR-gamma (PPARG) is abundantly expressed in fat cells and plays a key role in the formation of fat cells, in lipid metabolism and in the development of type 2 diabetes. PPARG knockout mice failed to develop normal adipose tissue and, when fed a high fat diet, displayed diminished weight gain and did not develop insulin resistance (Jones et al., PNAS 102: 6207-6212, 2005). The 12Ala variant is associated with a decrease in the binding affinity of the receptor with the PPAR response element in its target genes and thus with a reduction in its ability to regulate the expression of these target genes (Deeb et al., Nat Genet 20: 284-287, 1998). According to the 2006 obesity gene map (Rankinen et al., Obesity 14: 529-644), a total of 30 studies showed evidence of association between PPARG gene and obesity, and the majority of the positive findings involved the Pro12Ala polymorphism.

A large cross-sectional study, Quebec Family Study (QFS) (Robitaille et al., Clin Genet 63: 109-116, 2003) showed that subjects carrying the 12Pro allele were more responsive to the amount of fat in the diet. A similar study (Memisoglu et al., Human Molecular Genetics 12: 2923-2929, 2001) also showed that 12Pro/Pro subjects consuming high amounts of fat had a greater body mass index (BMI) than those consuming low amounts of fat. This association between dietary fat intake and BMI was not seen in 12Ala carriers, suggesting again that 12Pro/* subjects are more sensitive to the amount of fat in the diet. Strong evidence for genotypic differences in response to dietary intervention was obtained from the Finnish Diabetes Prevention Study (Lindi et al., Diabetes 51: 2581-2586, 2002). In response to a 3-year intervention involving diet and exercise, weight loss was greater in 12Ala/Ala subjects (-8.3 kg) than in Pro12Ala subjects (-4.0 kg) than in 12Pro/ Pro subjects (-3.4 kg). A study of overweight and obese women showed no differences in weight loss between 12Pro/Pro and 12Ala/* carriers in response to a 6-month low-calorie diet, but weight regain during follow-up (one year) was greater in women with the Ala allele than women homozygous for the 12Pro allele. In response to this intervention, Ala carriers exhibited greater increase in insulin sensitivity and fasting carbohydrate oxidation and greater decrease in fasting lipid oxidation (Nicklas et al., Diabetes 50: 2172-2176, 2001).

The 12Pro/Pro subjects (the most frequent genotype) are more sensitive to the amount of fat in the diet, more resistant to weight loss and at increased risk of diabetes. The evidence of gene-diet interaction is strong for this gene. Findings from diet intervention studies suggest a greater metabolic flexibility in the storage and mobilization of fat in 12Ala carriers, which is consistent with studies showing an increased BMI, a greater weight loss in response to intervention and a greater insulin sensitivity and reduced risk of diabetes. Thus, studies are consistent in showing that the 12Pro allele is the high-risk allele.

### ADRB2 rs 1042713 (G/A; Arg16Gly) and ADRB2 rs 1042714 (C/G; Gln27Glu) polymorphisms

The beta-2 adrenergic receptor (ADRB2) is the predominant form of the receptor expressed in fat cells, which plays a key role in breakdown of fat from the fat cells for energy in response to catecholamines. Several polymorphisms of this gene that result in amino acid changes have been identified, with the Arg16Gly and Gln27Glu polymorphisms being the most common ones in Caucasians, and those that have been most frequently investigated in relation to obesity. The two polymorphisms are in strong linkage disequilibrium (Meirhaeghe et al., Intntl J Obesity 24: 382-87, 2000). An in vitro study of recombinant expression of these receptors in Chinese hamster fibroblasts showed the functional impact of the two polymorphisms (Green et al., Biochemistry 33: 9414-9419, 1994). Compared to their respective normal alleles, the 16Gly allele was associated with enhanced downregulation of ADRB2 expression in response to agonist (isoproteranol) treatment, and 27Glu was associated with some increase (i.e., resistant to downregulation) in ADRB2 expression. Interestingly, the combination of both mutant alleles (16Gly and 27Glu) resulted in enhanced downregulation of receptor production. According to the recent obesity gene map (Rankinen et al., The human obesity gene map: The 2005 update. Obesity 14: 529-644), a total of 20 studies showed evidence of association between the ADRB2 gene and obesity, with most of the positive findings involving the Arg16Gly or Gln27Glu polymorphisms and some indication that the stronger association exists with the 27Glu allele. Some studies have demonstrated gender difference in risk for obesity with these polymorphisms (22. Hellstrom et al., J Intern Med 245: 253-259, 1999; Garenc et al., Obes Res 11: 612-618, 2003) but the preponderance of evidence does not favor making gender-specific genotype interpretations in this panel.

Multiple studies show evidence that the 27Glu allele was found to be positively associated with abdominal obesity (Lange et al., Int J Obes (Lond) 29: 449-457, 2005; Gonzalez et al., Clin Endocrinol (Oxf) 59: 476-481, 2003), as well as studies looking at both 27Glu and 16Gly alleles for risk of obesity and elevated fat mass (Masuo et al., Am J Hypertens, 19:1084-91, 2006). Longitudinal studies showed that weight gain from childhood to adulthood (Ellsworth et al. Int J Obes Relat Metab Disord 26: 928-937, 2002) and weight gain during adulthood (Masuo et al., Circulation 111: 3429-3434, 2005; van Rossum et al., Int J Obes Relat Metab Disord 26: 517-528, 2002) were higher in subjects who carried the 16Gly allele compared to the 16Arg/Arg subjects.

An increased risk of obesity (OR = 2.56) was found in 27Gln/Glu women having a high carbohydrate intake (CHO > 49% of total energy intake) while no association was observed in 27Gln/Gln women (Martinez et al., J Nutr 133: 2549-2554, 2003). In some cases, allelic interpretations for determining the best polymorphism and allele to make diet choices come from opposite intervention (overfeeding) studies and choice of the opposing allele. For example, the results from an overfeeding study (an extra 1000 kcal/day for 100 days) performed in pairs of male identical twins showed that 27Gln/Gln subjects gained more weight and subcutaneous fat than carriers of the 27Glu allele (Ukkola et al., Int J Obes Relat Metab Disord 25: 1604-1608, 2001). In a study of overweight Japanese men enrolled in a 24-month weight loss program (low-calorie diet (1,600 kcal/day) and aerobic exercise one hour daily) showed a higher frequency of the 16Gly allele in men resistant to weight loss (defined as BMI change less than 10%; n = 81) and those who regained body weight after successful initial weight loss at 6 months (Masuo et al., Circulation 111: 3429-3434, 2005). Women who were more active during their leisure time and were carriers of the 27Glu allele had higher BMI compared to non-carriers, suggesting that these women may be more resistant to losing weight (Corbalan et al., Clin Genet 61: 305-307,2002).

### ADRB3 rs4994 (C/T; Arg64Trp) polymorphism

The adrenergic beta-3 receptor (ADRB3) is involved in the regulation of lipolysis in white adipose tissue, and is mainly expressed in visceral adipose tissue, the fat depot that is closely associated with the obesity-related metabolic complications. In vitro studies on isolated adipocytes showed that the mutation results in a deterioration of lipolysis in response to a specific agonist in cells carrying the 64Arg allele (Umekawa et al., Diabetes 48: 117-120, 1999). A haplotype formed of three variants in the ADRB3 gene, including the 64Arg variant, was found to be associated with increased BMI (n=208) and with a 10-fold decrease in the sensitivity (induced lipolysis) of visceral adipocytes to a selective β3-receptor agonist (Hoffstedt et al., Diabetes 48: 203-205, 1999). The three variants are in linkage disequilibrium, which suggests that the 64Arg variant is associated with reduced receptor function. A total of 29 studies showed evidence of association between the ADRB3 gene and obesity. One meta-analysis based on 31 studies with more than 9,000 subjects showed a higher BMI (0.30 kg/m² higher on average) in carriers of the 64Arg variant compared to homozygous 64Trp/Trp subjects (Fujisawa et al., J Clin Endocrinol Metab 83: 2441-2444, 1998). A second one based on more than 6,500 subjects (mainly Japanese subjects) from 22 studies also showed higher BMI values in carriers of the 64Arg variant (0.26 kg/m² higher on average) compared to non-carriers (Kurokawa et al., Obes Res 9: 741-745, 2001).

A case-control study (158 obese, 154 normal weight) showed an increased risk of obesity (OR = 2.98) in 64Arg carriers (higher BMI) only among sedentary subjects, but not in physically active subjects where genotypic differences in BMI were not found (Marti et al., Diabetes Obes Metab 4: 428-430, 2002), A study of 61 obese women with type 2 diabetes who submitted to a 3-month intervention combining low-calorie diet and exercise showed that women with the 64Arg variant lost less weight (4.6 kg vs 8.3 kg) and body mass (1.9 kg/m2 vs 3.4 kg/m2) than 64Trp/Trp women (Sakane et al., Diabetes Care 20: 1887-1890, 1997). A study performed in 76 perimenopausal women who submitted to a 3-month intervention combining exercise and diet found that 48% of the women with the 64Arg variant lost weight compared to 69% of the women without the variant (Shiwaku et al., Int J Obes Relat Metab Discord 27: 1028-1036, 2003). These two studies suggest that the variant is associated with difficulty in losing weight through diet and exercise. A study (Phares et al., Obes Res 12: 807-815, 2004) performed on 29 men and 41 women showed that ADRB3 64Arg carriers experienced greater loss of fat mass and trunk fat following 24 weeks of supervised aerobic exercise training compared to non-carriers. These results seem to demonstrate an opposite allelic response to exercise, but the level of exercise in this study regimen was more vigorous, supervised endurance training. Interpretation of genotypic differences in response to exercise may be further complicated in many studies because the obese state may be a confounding factor masking moderate effects of the variant on energy expenditure (Tchernof et al., Diabetes 48:1425-1428,1999).

Thus, we disclose a method for identifying a subject's metabolic genotype comprising identifying the subject's genotype with respect to one or more (*i.e.,* 2, 3, or 4) of the FABP2 locus, PPARG locus, ADRB3 locus, and/or ADRB2 locus.

A method for identifying a subject's metabolic genotype comprises identification of the subject's genotype with respect to accessing the subject's genotype with one or more (*i.e.,* 2, 3, 4, or 5) of the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

We disclose a method for identifying a subject's single polymorphism metabolic genotype comprising identification of the genotype with respect to a metabolic gene allele selected from the group consisting of FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

A method for identifying a subject's composite metabolic genotype comprises identification of the genotype with respect to at least two metabolic gene alleles selected from the group consisting of FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

A method for identifying a subject's metabolic genotype comprises identification of the composite polymorphism genotype with respect to at least three metabolic gene alleles selected from the group consisting of FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

The invention provides a method for identifying a subject's metabolic genotype comprising identification of the composite polymorphism genotype with respect to at least four metabolic gene alleles selected from the group consisting of FABP2 (rs1799883; G/A) locus, PPARG (rs1801282; C/G) locus, ADRB3 (rs4994; C/T) locus, ADRB2 (rs1042713; AiG) locus, and/or ADRB2 (rs1042714; C/G) locus. In particular, the method of the invention comprises determining the genotype with respect to the FABP2 (rs 179983; G/A), PPARG (rs1801282; C/G); and ADRB2 (rs1042714; C/G) loci and at least one of the ADRB3 (rs4994; C/T) locus and the ADRB2 (rs1042713; A/G) locus.

According to some embodiments there is provided a method for identifying a subject's metabolic genotype comprising identifying the composite polymorphism genotype with respect to each of the metabolic gene alleles FABP2 (rs1799883; G/A) locus, PPARG (rs1801282;C/G locus, ADRB3 (rs4994; C/T) locus, ADRB2 (rs1042713; A/G) locus, and/or (rs1042714; C/G) locus.

A subject's single polymorphism metabolic genotype and/or composite metabolic genotype results may be classified according to their relationships to weight management risk, including what constitutes a "less responsive" or "more responsive" result from diet and/or exercise interventions, 2) their associated clinical or health-related biomarker outcomes, 3) their relationships to intervention choices for weight management, and 4) prevalence of each genotype. Table 1 and 2 below defines the alleles of certain metabolic genes and explains the increased risk for susceptibility to certain metabolic disorders/parameters.

**TABLE 1: Subject Metabolic Gene/Polymorphism**

| **GENE** | **Locus/SNP** | **GENOTYPE** | **Pop. Freq *** |
|---|---|---|---|
| **FABP2** | FABP2 (+54) | 1.2 or 2.2 | 48% |
| | | G/A or A/A | |
| | Ala54Thr | (54Ala/Thr or 54Thr/Thr) | |
| | Ala = G= allele 1 | 1.1 | 52% |
| | Thr = A= allele 2 | G/G | |
| | rs1799883 | (54 Ala/Ala) | |
| **PPARG** | PPARG (+12) | 1.1 | 81% |
| | | C/C | |
| | Pro12Ala | (12Pro/Pro) | |
| | Pro = C = allele 1 | 1.2 or 2.2; | 19% |
| | Ala = G = allele 2 | C/G or G/G | |
| | rs 1801282 | (12Pro/Ala or 12Ala/Ala) | |
| **ADRB2** | ADRB2 (+27) | 1.2 or 2.2 | 63 % |
| | | C/G or G/G | |
| | Gln27Glu | (27Gln/Glu or 27Glu/Glu) | |
| | Gln = C= allele 1 | 1.1 | 37% |
| | Glu = G= allele 2 | C/C | |
| | rs1042714 | (27Gln/Gln) | |
| **ADRB2** | ADRB2 (+16) | 1.1 or 1.2 | 86% |
| | | G/G or G/A | |
| | Arg16Gly | (16Gly/Gly or 16Gly/Arg) | |
| | Gly = G= allele 1 | 2.2 | 14% |
| | Arg = A= allele 2 | A/A | |
| | rs 1042713 | (16Arg/Arg) | |
| **ADRB3** | ADRB3 (+64) | 1.2 or 2.2 | 16% |
| | | T/C or C/C | |
| | Arg64Trp | (64Trp/Arg or 64Arg/Arg) | |
| | Trp = T= allele 1 | 1.1 | 84% |
| | Arg = C= allele 2 | T/T | |
| | rs4994 | (64Trp/Trp) | |

| | | | |
|---|---|---|---|
| * Pop. Freq = population frequency, determined for Caucasians using Quebec Family Study (QFS) database | | | |

**TABLE 2: Subject Susceptibility Chart Based on Metabolic Genotype**

| **Genotype** | **Disease Risk** | **Biomarker Risk **** | **Actionable Information***** |
|---|---|---|---|
| FABP2 (+54; rs1799883) 1.2 or 2.2 | Obesity | ↑BMI | Subjects with this genotype have an enhanced absorption of dietary fat and a slower metabolism, which result in a greater propensity for weight gain and a decreased ability to lose weight. Clinical studies indicate subjects with this genotype will improve their risks of elevated triglycerides, insulin and blood sugars by reducing saturated fat and trans fat, and increasing monounsaturated fats while moderating carbohydrate in the diet. |
| | Insulin | ↑Body fat | |
| | Resistance | ↑Abd fat | |
| | Metabolic | ↑TGs | |
| | Syndrome | ↑Insulin | |
| | | ↑BS | |
| | | ↑TNFα | |
| | | ↓RMR | |
| FABP2 (+54; rs1799883) 1.1 | Negative | No | Subjects with this genotype have normal absorption of dietary fat. Clinical studies have demonstrated these subjects respond to a low calorie, low fat diet with weight loss; decreased body fat, and lower LDL cholesterol levels. |
| PPARG (+12; rs1801282) 1.1 | Obesity Diabetes | ↑BMI | PPARG plays a key role in fat cell formation and fat metabolism. Clinical studies indicate subjects with this genotype have a high risk of weight gain and are less responsive to the effect of a low calorie diet on weight loss. Those with a high total fat and polyunsaturated fat intake tend to have a significantly higher BMI than the alternative genotype. |
| | | ↑Abd fat | |
| | | ↓HDL | |
| PPARG (+12; rs1801282) 1.2 or 2.2 | Obesity | ↑BMI | Subjects with this variant have variations in fat cell formation and fat metabolism that increase their sensitivity to the effects of changes in diet. These subjects have an easier time losing weight from a low calorie diet; however, they are at risk to regain it. Women are 5 fold more likely than the alternative genotype to be obese if their habitual carbohydrate intake exceeds 49%. Therefore, modulation of carbohydrate intake will be beneficial to these subjects to prevent their risk of obesity. They do have a higher BMI as a result of a high saturated and low monounsaturated fat intake. Therefore, the quality of fat in their diet is also important. |
| ADRB2 (+27; rs1042714) 1.2 or 2.2 | Obesity Diabetes | ↑BMI | Subjects with this gene variant are less able to mobilize their fat stores for energy. Women with this variant have 2½ times the risk of obesity and elevated insulin levels if their habitual carbohydrate intake exceeds 49% of total calories when compared to subjects with the alternative genotype. Modulation of carbohydrate intake has been shown to reduce insulin levels and will be beneficial to these subjects to prevent their risk of obesity and elevated triglycerides. Both men and women with this genotype are more resistant to the weight loss effect of a low calorie diet and aerobic exercise. |
| | | ↑Abd fat | |
| | | ↑TGs | |
| | | ↑Insulin | |
| | | ↑BS | |
| ADRB2 (+27; rs1042714) 1.1 | Negative | No | Subjects with this genotype have a normal breakdown of fat for energy. Consuming a high intake of dietary carbohydrates shows no specific effect on body weight. Men who engage in regular physical activity have a significantly reduced obesity risk. Overall, subjects with this genotype are likely to respond with weight change and improvement in health outcomes from changes in diet and aerobic exercise. |
| ADRB2 (+16; rs1042713) 1.1 or 1.2 | Obesity | ↑BMI | Subjects with this gene variant are less able to mobilize their fat stores for energy in response to a physiologic stress, such as exercise. As a result, they mobilize less cellular fat and lose less weight and body fat than expected in response to aerobic exercise. Additionally, they are at greater risk of rebound weight gain. |
| | | ↑Body fat -Men | |
| | | ↓Body fat-Women | |
| ADRB2 (+16; rs1042713) 2.2 | Negative | No | Subjects with this genotype mobilize fat from their fat cells for energy effectively as a result of a low calorie diet and aerobic exercise for weight loss. They are more likely to lose the body weight and fat and to keep it off. |
| ADRB3 (+64; rs4994) 1.2 or 2.2 | Obesity | ↑BMI | Subjects with this genotype do not break down abdominal fat for energy in response to a physiologic stress, such as exercise. As a result, they have a slower energy metabolism and are not so responsive to the beneficial effects of aerobic exercise (weight loss, loss of abdominal fat). |
| | DM | ↑Abd fat | |
| | | ↓RMR | |
| ADRB3 (+64; rs4994) 1.1 | Negative | No | Subjects with this genotype have a normal metabolic rate and breakdown of abdominal body fat. Studies have shown these subjects experience weight loss by engaging in light to moderate aerobic exercise. |

| | | | |
|---|---|---|---|
| ** BMI = body mass index, TGs = triglycerides, abd fat = abdominal fat, BS = blood sugars, TNFα= tumor necrosis factor alpha, RMR = resting metabolic rate, HDL = high density lipoprotein. *** Metabolism, nutrition and exercise implications. | | | |

Methods and kits are disclosed for the measurement of blood lipid levels in a subject for selecting or screening subjects for appropriate therapeutic or dietary intervention or lifestyle change.

The subject's HDL, LDL and/or triglycerides are measured. The subject is considered to have an abnormal lipid profile or dyslipidemia when screened as having lower level of HDL, about 40 mg/dL or lower for men, and 50 mg/dL or lower for women, or higher level of LDL, about 100 mg/dL or above, or higher level of triglycerides, about 150 mg/dL or above, or any combination thereof.

Lower level of HDL is 20-60 mg/dL or 50-59 mg/dL or 40-49 mg/dL or 30-39 mg/dL or <30 mg/dL; higher level of LDL is 100->190 mg/dL or 100-129 mg/dL or 130-159 mg/dL or 160-190 mg/dL or >190 mg/dL; and higher level of triglyceride is 150- >500 mg/dL or 150-199 mg/dL or 200-500 mg/dL or >500 mg/dL.

Subjects may be screened for clinical trials for response to weigh-management strategy, or therapeutic interventions, comprising identifying subjects by their allelic profile and/or composite genotypes of this invention and predicting for their response to recommended therapy/diet/lifestyle or combination thereof, with their predicted levels of HDL, or LDL or triglycerides.

Methods and kits for screening subjects for clinial trials for weight management are described, wherein an underweight subject has a BMI <18.5; an overweight subject in the range 25-29.9, an obese subject has a BMI of 30-39.9, and BMI of >40.0 is considered extremely obese. Identification of metabolic genotype in these subjects could provide health professionals with tools to discuss about the difficulties of a subject with a BMI of 25 to reach BMI of 22 with a lower-calorie diet alone.

Table 3 provides the ethnic prevalence for certain metabolic genotypes.

**TABLE 3: Prevalence of the Genotype/Risk (‡) Patterns by Ethnicity**

| Gene/Genotype Result | Caucasian (QFS) | Black | Hispanic | Japanese | Chinese | Korean |
|---|---|---|---|---|---|---|
| FABP2 rs1799883 1.2 or 2.2 ‡ | 48% | 35% | 59% | 58% | 54% | 55% |
| FABP2 rs1799883 1.1 | 52% | 65% | 41% | 42% | 46% | 45% |
| PPARG rs1801282 1.1 ‡ | 81% | 96% | 82% | 92% | 95% | 90% |
| PPARG rs1801282 1.2 or 2.2 | 19% | 4% | 18% | 8% | 5% | 10% |
| ADRB2 rs1042714 1.2 or 2.2 ‡ | 63% | 35% | 59% | 12-18% | 41-59% | 21% |
| ADRB2 rs1042714 1.1 | 37% | 65% | 41% | 82-88% | 41-59% | 79% |
| ADRB2 rs1042713 1.1 or 1.2 ‡ | 86% | 74-80% | 70-81% | 71-81% | 63-73% | 61% |
| ADRB2 rs1042713 2.2 | 14% | 20-26% | 19-30% | 19-29% | 27-37% | 39% |
| ADRB3 rs4994 1.2 or 2.2 ‡ | 16% | 19-27% | 20-35% | 33% | 24-32% | 28% |
| ADRB3 rs4994 1.1 | 84% | 73-81% | 65-80% | 67 % | 68-76% | 72% |

| | | | | | | |
|---|---|---|---|---|---|---|
| ‡ = Indicates risk genotype(s) | | | | | | |

Combinations of these gene variations affect 1) how subjects respond to specific macronutrients in their diet and 2) their different tendencies in energy metabolism that ultimately influence their ability to maintain or lose weight through exercise. A metabolic genotype determination will help healthy subjects identify a genetic risk for adverse weight management issues that have not yet manifested. Knowing gene-related risks early can assist in making personalized health decisions (nutrition, lifestyle) to preserve future health, as well as provide direction on how best to prioritize a subject's focus on nutrition and lifestyle choices to manage optimal body weight and body composition.

Information learned from a subject's metabolic genotype may be used to predict a subject's genetic risk for adverse weight management issues. The subject's genotype may be used to assess risk and allow for the selection of an appropriate therapeutic/dietary regimen or lifestyle recommendation. Identifying a subjecting genotype may be used to pairing the subject with a therapeutic or nutrition or lifestyle alteration or a combination of any two or three to devise a strategy to achieve and/or sustain weight loss. Generally, a subject's allelic pattern of one or more metabolic genes may be used to classify the subject's predicted responsiveness to macronutrients and energy restriction in the diet, with or without exercise, in a weight loss management program. Accordingly, a personalized weight management program may be selected for the subject based on subject's predicted response. For example, a weight management program may classify a subject's metabolic genotype into one of a series of nutrition categories and one of a series of exercise categories based upon that subject's predisposition for responsiveness to certain macronutrients and degree of exercise. The nutrition category, exercise category, or combination thereof may be selected for a subject based on subject's genetic patterns.

According to some embodiments, a method is provided for selecting an appropriate therapeutic/dietary regimen or lifestyle recommendation for a subject comprising: determining a subject's genotype with respect to the polymorphic loci FABP2 (rs1799883; G/A) locus, PPARG (rs1801282;C/G) locus and ADRB2 (rs1042714; C/G) locus, and one or both of the ADRB3 (rs4994; C/T) locus and ADRB2 (rs1042713; A/G) locus wherein the subject's genotype with respect to said loci provides information about the subject's increased susceptibility to adverse weight management issues, and allows the selection of a therapeutic/dietary regiment or lifestyle recommendation that is suitable to the subject's susceptibility to adverse weight management issues.

The subject with a combined genotype of FABP2 (rs1799883) 1.1, PPARG (rs1801282) 1.1, ADRB2 (rs1042714) 1.1, and ADRB2 (rs1042713) 2.2, and ADRB3 (rs4994) 1.1 is predicted to be responsive to: a low fat or low carbohydrate, calorie-restricted diet; regular exercise; or both.

A subject with a combined genotype of one of FABP2 (rs1799883) 1.1 or 1.2 and PPARG (rs1801282) 1.1, and additionally one of ADRB2 (rs1042714) 1.1, 1.2, or 2.2 in combination with ADRB2 (rs1042713) 2.2 and ADRB3 (rs4994) 1.1 is predicted to be responsive to: a low fat, calorie-restricted diet; regular exercise; or both.

A subject with a combined genotype of one of PPARG (rs1801282) 1.2 or 2.2 and/or one of ADRB2 (rs1042714) 1.2 or 2.2, in combination with ADRB2 (rs1042713) 2.2 and ADRB3 (rs4994) 1.1 is predicted to be responsive to: a low carbohydrate, calorie-restricted diet; regular exercise; or both.

A subject with a combined genotype of one of PPARG (rs1801282) 1.2 or 2.2 and one of FABP2 (rs1799883) 1.1 or 1.2, in combination with ADRB2 (rs1042713) 2.2 and ADRB3 (rs4994) 1.1 is predicted to be responsive to: a low carbohydrate, calorie-restricted diet; regular exercise; or both.

A subject with a combined genotype of FABP2 (rs1799883) 1.1 and PPARG (rs1801282) 1.1, in combination with one of ADRB2 (rs1042713) 1.2 or 1.1 or one of ADRB3 (rs4994) 1.2 or 2.2 is predicted to be responsive to a low fat or low carbohydrate, calorie-restricted diet. Optionally, the subject is further predicted to be less responsive to regular exercise.

A subject with a combined genotype of one of FABP2 (rs1799883) 1.1 or 1.2 and PPARG (rs1801282) 1.1, in combination with one of ADRB2 (rs1042714) 1.1, 1.2, or 2.2 and either one of ADRB2 (rs1042713) 1.1 or 1.2 or one of ADRB3 (rs4994) 1.2 or 2.2 is predicted to be responsive to: a low fat, calorie-restricted diet. Optionally, the subject is further predicted to be less responsive to regular exercise.

A subject with a combined genotype of one of PPARG (rs1801282) 1.2 or 2.2 and/or one of ADRB2 (rs1042714) 1.2 or 2.2, in combination with one of ADRB2 (rs1042713) 1.1 or 1.2 or one of ADRB3 (rs4994) 1.2 or 2.2 is predicted to be responsive to: a low carbohydrate, calorie-restricted diet. Optionally, the subject is further predicted to be less responsive to regular exercise.

A subject with a combined genotype of one of PPARG (rs1801282) 1.2 or 2.2 and one of FABP2 (rs1799883) 1.1 or 1.2, in combination with one of ADRB2 (rs1042713) 1.1 or 1.2 or one of ADRB3 (rs4994) 1.2 or 2.2 is predicted to be responsive to: a low carbohydrate, calorie-restricted diet. Optionally, the subject is further predicted to be less responsive to regular exercise.

According to some embodiments, the therapeutic/dietary regimen comprises of administering a nutraceutical.

Optionally, the methods above further comprise classifying the subject with respect to likely benefit from a therapeutic/dietary regimen or lifestyle change.

According to some embodiments, the low fat diet of the methods described above provide no more than about 35 percent of total calories from fat.

According to some embodiments, the low carbohydrate diet of the methods described above provide less than about 50 percent of total calories from carbohydrates.

According to some embodiments, the calorie-restricted diet of the methods described above restrict total calories to less than 95% of the subject's weight management level.

We disclose a method for identifying a subject's metabolic genotype comprising: identifying the subject's genotype with respect to at least three of the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

According to some embodiments, a method is provided for identifying a subject's metabolic genotype comprising: identifying the subject's genotype with respect to the FABP2 (rs1799883; G/A) locus, PPARG (rs1801282; C/G) locus, and ADRB2 (rs1042714; C/G) locus, plus at least one of the ADRB3 (rs4994; C/T) locus and ADRB2 (rs1042713; A/G) locus.

According to some embodiments, methods are provided for selecting an appropriate therapeutic/dietary regimen or lifestyle recommendation for a subject comprising: a) determining a subject's genotype with respect to at least four of the polymorphic loci, selected from: FABP2 (rs1799883; G/A) locus; PPARG (rs1801282; C/G) locus; and ADRB2 (rs1042714; C/G) locus; and at least one of the ADRB3 (rs4994: C/T locus; and ADRB2 (rs1042713; A/G) locus; and b) classifying the subject into a nutrition category and/or an exercise category for which the subject is predicted to obtain a likely benefit, wherein the nutrition category is selected from a low fat diet; a low carbohydrate diet; a high protein diet; and a calorie restricted diet, and wherein the exercise category is selected from: light exercise; normal exercise; and vigorous exercise.

We disclose a method for selecting an appropriate therapeutic/dietary regimen or lifestyle recommendation for a subject comprising: (a) detecting an allelic pattern of at least two alleles selected from the group consisting of FABP2 (rs1799883) allele 1 (Ala or G), FABP2 (rs1799883) allele 2 (Thr or A), PPARG (rs1801282) allele 1 (Pro or C), PPARG (rs1801282) allele 2 (Ala or G), ADRB3 (rs4994) allele 1 (Trp or T), ADRB3 (rs4994) allele 2 (Arg or C), ADRB2 (rs1042713) allele 1 (Gly or G), ADRB2 (rs1042713) allele 2 (Arg or A), ADRB2 (rs1042714) allele 1 (Gln or C) and ADRB2 (rs1042714) allele 2 (Glu or G), wherein the presence of the allelic pattern is predictive of the subject's response to diet and/or exercise and (b) selecting a therapeutic/dietary regimen or lifestyle recommendation that is suitable for the subject's predicted response to diet and/or exercise.

According to some embodiments, a subject with a combined genotype of FABP2 (rs1799883) 1.1 (Ala/Ala or G/G), PPARG (rs1801282) 1.1 (Pro/Pro or C/C), ADRB2 (rs1042714) 1.1 (Gln/Gln or C/C), and ADRB2 (rs1042713) 2.2 (Arg/Arg or A/A), and ADRB3 (rs4994) 1.1 (Trp/Trp or T/T) is predicted to be responsive to: a low fat or low carbohydrate, calorie-restricted diet; regular exercise; or both.

According to some embodiments, a subject with a combined genotype of one of FABP2 (rs1799883) 1.1 (Ala/Ala or G/G) or 1.2 (Ala/Thr or G/A) and PPARG (rs1801282) 1.1 (Pro/Pro or C/C), and additionally one of ADRB2 (rs1042714) 1.1 (Gln/Gln or C/C), 1.2 (Gln/Glu or C/G), or 2.2 (Glu/Glu or G/G) in combination with ADRB2 (rs1042713) 2.2 (Arg/Arg or A/A) and ADRB3 (rs4994) 1.1 (Trp/Trp or T/T) is predicted to be responsive to: a low fat, calorie-restricted diet; regular exercise; or both.

According to some embodiments, a subject with a combined genotype of one of PPARG (rs1801282) 1.2 (Pro/Ala (C/G) or 2.2 (Ala/Ala or G/G) and/or one of ADRB2 (rs1042714) 1.2 (Gln/Glu or C/G) or 2.2 (Glu/Glu or G/G), in combination with ADRB2 (rs1042713) 2.2 (Arg/Arg or A/A) and ADRB3 (rs4994) 1.1 (Trp/Trp or T/T) is predicted to be responsive to: a low carbohydrate, calorie-restricted diet; regular exercise; or both.

According to some embodiments, a subject with a combined genotype of one of PPARG (rs1801282) 1.2 (Pro/Ala or C/G) or 2.2 (Ala/Ala or G/G) and one of FABP2 (rs1799883) 1.1 (Ala/Ala or G/G) or 1.2 (Ala/Thr or G/A), in combination with ADRB2 (rs1042713) 2.2 (Arg/Arg or A/A) and ADRB3 (rs4994) 1.1 (Trp/Trp or T/T) is predicted to be responsive to: a low carbohydrate, calorie-restricted diet; regular exercise; or both.

A subject with a combined genotype of FABP2 (rs1799883) 1.1 (Ala/Ala or G/G) and PPARG (rs1801282) 1.1 (Pro/Pro or C/C), in combination with one of ADRB2 (rs1042713) 1.2 (Gly/Arg or G/A) or 2.2 (Arg/Arg or A/A) or one of ADRB3 (rs4994) 1.2 (Arg/Trp or T/C) or 2.2 (Arg/Arg or C/C) is predicted to be responsive to a low fat or low carbohydrate, calorie-restricted diet. Optionally, the subject is further predicted to be less responsive to regular exercise.

According to some embodiments, a subject with a combined genotype of one of FABP2 (rs1799883) 1.1 (Ala/Ala or G/G) or 1.2 (Ala/Thr or G/A) and PPARG (rs1801282) 1.1 (Pro/Pro or C/C), in combination with one of ADRB2 (rs1042714) 1.1 (Gln/Gln or C/C), 1.2 (Gln/Glu or C/G), or 2.2 (Glu/Glu or G/G) and either one of ADRB2 (rs1042713) 1.1 (Gly/Gly or G/G) or 1.2 (Gly/Arg or G/A) or one of ADRB3 (rs4994) 1.2 (Trp/Arg or T/C) or 2.2 (Arg/Arg or C/C) is predicted to be responsive to: a low fat, calorie-restricted diet. Optionally, the subject is further predicted to be less responsive to regular exercise.

A subject with a combined genotype of one of PPARG (rs1801282) 1.2 (Pro/Ala or C/G) or 2.2 (Ala/Ala or G/G) and/or one of ADRB2 (rs1042714) 1.2 (Gln/Glu or C/G) or 2.2 (Glu/Glu or G/G), in combination with one of ADRB2 (rs1042713) 1.1 (Gly/Gly or G/G) or 1.2 (Gly/Arg or G/A) or one of ADRB3 (rs4994) 1.2 (Trp/Arg or T/C) or 2.2 (Arg/Arg or C/C) is predicted to be responsive to: a low carbohydrate, calorie-restricted diet. Optionally, the subject is further predicted to be less responsive to regular exercise.

A subject with a combined genotype of one of PPARG (rs1801282) 1.2 (Pro/Ala or C/G) or 2.2 (Ala/Ala or G/G) and one of FABP2 (rs1799883) 1.1 (Ala/Ala or G/G) or 1.2 (Ala/Thr or G/A), in combination with one of ADRB2 (rs 1042713) 1.1 (Gly/Gly or G/G) or 1.2 (Gly/Arg or G/A) or one of ADRB3 (rs4994) 1.2 (Trp/Arg or T/C) or 2.2 (Arg/Arg or C/C) is predicted to be responsive to: a low carbohydrate, calorie-restricted diet. Optionally, the subject is further predicted to be less responsive to regular exercise.

We describe a method for predicting a subject's genetic risk for adverse weight management issues comprising: detecting a genetic polymorphism pattern comprising at least two alleles selected from the group consisting of FABP2 (rs1799883) allele 1 (Ala or G), FABP2 (rs1799883) allele 2 (Thr or A), PPARG (rs1801282) allele 1 (Pro or C), PPARG (rs1801282) allele 2 (Ala or G), ADRB3 (rs4994) allele 1 (Trp or T), ADRB3 (rs4994) allele 2 (Arg or C), ADRB2 (rs1042713) allele 1 (Gly or G), ADRB2 (rs1042713) allele 2 (Arg or A), ADRB2 (rs1042714) allele 1 (Gln or C) and ADRB2 (rs1042714) allele 2 (Glu or G), wherein the presence of the genetic polymorphism pattern is predictive of the subject's response to diet and/or exercise.

Optionally, the therapeutic/dietary regimen comprises administering a nutraceutical.

Optionally, the methods above further comprise classifying the subject with respect to likely benefit from a therapeutic/dietary regimen or lifestyle change.

Optionally, the low fat diet of the methods described above provide no more than about 35 percent of total calories from fat.

Optionally, the low carbohydrate diet of the methods described above provide less than about 50 percent of total calories from carbohydrates.

Optionally, the calorie-restricted diet of the methods described above restrict total calories to less than 95% of the subject's weight management level.

We describe kits comprising: a) reagents for determining a subject's genotype with respect to any four of the polymorphic loci, selected from the following: FABP2 (rs1799883; G/A) locus; PPARG (rs1801282; C/G) locus; ADRB3 (rs4994; C/T) locus; ADRB2 (rs1042713; A/G) locus; and ADRB2 (rs1042714; C/G) locus; and b) instructions for determining the subject's metabolic genotype, and means for classifying the subject into a nutrition category and/or an exercise category for which the subject is predicted to obtain a likely benefit, wherein the nutrition category is selected from the group consisting of a low fat diet; a low carbohydrate diet; a high protein diet; and a calorie restricted diet, and wherein the exercise category is selected from the group consisting of: light exercise; normal exercise; and vigorous exercise.

Optionally, the kit further classifies the subject with respect to likely benefit from a therapeutic/dietary regimen or lifestyle change.

Optionally, the kit comprises reagents for genotyping a subject for a combined genotype of FABP2 (rs1799883) 1.1, PPARG (rs1801282) 1.1, ADRB2 (rs1042714) 1.1, and ADRB2 (rs1042713) 2.2, and ADRB3 (rs4994) 1.1 is predicted to be responsive to: a low fat or low carbohydrate, calorie-restricted diet; regular exercise; or both.

Optionally, the kit comprises reagents for genotyping a subject for a combined genotype of one of FABP2 (rs1799883) 1.1 or 1.2 and PPARG (rs1801282) 1.1, and additionally one of ADRB2 (rs1042714) 1.1, 1.2, or 2.2 in combination with ADRB2 (rs1042713) 2.2 and ADRB3 (rs4994) 1.1 is predicted to be responsive to: a low fat, calorie-restricted diet; regular exercise; or both.

We describe a kit comprising reagents for genotyping a subject with a combined genotype of one of PPARG (rs1801282) 1.2 or 2.2 and/or one of ADRB2 (rs1042714) 1.2 or 2.2, in combination with ADRB2 (rs1042713) 2.2 and ADRB3 (rs4994) 1.1 is predicted to be responsive to: a low carbohydrate, calorie-restricted diet; regular exercise; or both.

Optionally, the kit comprises reagents for genotyping a subject for a combined genotype of one of PPARG (rs1801282) 1.2 or 2.2 and one of FABP2 (rs1799883) 1.1 or 1.2, in combination with ADRB2 (rs1042713) 2.2 and ADRB3 (rs4994) 1.1 is predicted to be responsive to: a low carbohydrate, calorie-restricted diet; regular exercise; or both.

Optionally, the kit comprises reagents for genotyping a subject for a combined genotype of FABP2 (rs1799883) 1.1 and PPARG (rs1801282) 1.1, in combination with one of ADRB2 (rs1042713) 1.2 or 1.1 or one of ADRB3 (rs4994) 1.2 or 2.2 is predicted to be responsive to a low fat or low carbohydrate, calorie-restricted diet.

Optionally, the kit comprises reagents for genotyping a subject for a combined genotype of one of FABP2 (rs1799883) 1.1 or 1.2 and PPARG (rs1801282) 1.1, in combination with one of ADRB2 (rs1042714) 1.1, 1.2, or 2.2 and either one of ADRB2 (rs1042713) 1.1 or 1.2 or one of ADRB3 (rs4994) 1.2 or 2.2 is predicted to be responsive to: a low fat, calorie-restricted diet.

Optionally, the kit comprises reagents for genotyping a subject for a combined genotype of one of PPARG (rs1801282) 1.2 or 2.2 and/or one of ADRB2 (rs1042714) 1.2 or 2.2, in combination with one of ADRB2 (rs1042713) 1.1 or 1.2 or one of ADRB3 (rs4994) 1.2 or 2.2 is predicted to be responsive to: a low carbohydrate, calorie-restricted diet.

Optionally, the kit comprises reagents for genotyping a subject for a combined genotype of one of PPARG (rs1801282) 1.2 or 2.2 and one of FABP2 (rs1799883) 1.1 or 1.2, in combination with one of ADRB2 (rs1042713) 1.1 or 1.2 or one of ADRB3 (rs4994) 1.2 or 2.2 is predicted to be responsive to: a low carbohydrate, calorie-restricted diet.

Optionally, kits comprise: reagents and instructions for determining a subject's metabolic genotype, comprising: identifying the subject's genotype with respect to at least four of the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

Optionally, kits comprise: reagents and instructions for determining a subject's metabolic genotype, comprising: identifying the subject's genotype with respect to at least three of the FABP2 (rs1799883; G/A) locus, PPARG (rs1801282; C/G) locus, ADRB3 (rs4994; C/T) locus, ADRB2 (rs1042713; A/G) locus, and/or ADRB2 (rs1042714; C/G) locus.

### Nutrition categories

Nutrition categories are generally classified on the basis of the amount of macronutrients (i.e., fat, carbohydrates, protein) recommended for a subject based on that subject's metabolic genotype. The primary goal of selecting an appropriate therapeutic/dietary regimen or lifestyle recommendation for a subject is to pair a subject's metabolic genotype with the nutrition category to which that subject is most likely to be responsive. A nutrition category is generally expressed in terms of the relative amounts of macronutrients suggested for a subject's diet or in terms of calories restrictions (*e.g.,* restricting the total number of calories a subject receives and/or restricting the number of calories a subject receives from a particular macronutrient). For example, nutrition categories may include, but are not limited to, 1) low fat, low carbohydrate diets; 2) low fat diets, or 3) low carbohydrate diets. Alternatively, nutrition categories may be classified on the basis of the restrictiveness of certain macronutrients recommended for a subject based on that subject's metabolic genotype. For example, nutrition categories may be expressed as 1) balanced or calorie restricted diets; 2) fat restrictive diets, or 3) carbohydrate restrictive diets.

Subjects with a metabolic genotype that is responsive to fat restriction or low fat diet tend to absorb more dietary fat into the body and have a slower metabolism. They have a greater tendency for weight gain. Clinical studies have shown these subjects have an easier time reaching a healthy body weight by decreasing total dietary fat. They may have greater success losing weight by following a reduced fat and/or reduced calorie diet. In addition, they benefit from replacing saturated fats with monounsaturated fats within a reduced calorie diet. Clinical studies have also shown these same dietary modifications improve the body's ability to metabolize sugars and fats.

Subjects with a metabolic genotype that is responsive to carbohydrate restriction or low carbohydrate diet tend to be more sensitive to weight gain from excessive carbohydrate intake. They may have greater success losing weight by reducing carbohydrates within a reduced calorie diet. Subjects with this genetic pattern are prone to obesity and have difficulty with blood sugar regulation if their daily carbohydrate intake is high, such as where the daily carbohydrate intake exceeds, for example, about 49% of total calories. Carbohydrate reduction has been shown to optimize blood sugar regulation and reduce risk of further weight gain. If they have high saturated and low monounsaturated fats in their diet, risk for weight gain and elevated blood sugar increases. While limiting total calories, these subjects may benefit from restricting total carbohydrate intake and shifting the fat composition of their diet to monounsaturated fats (*e.g.,* a diet low in saturated fat and low in carbohydrate).

Subjects with a metabolic genotype that is responsive to a balance of fat and carbohydrate show no consistent need for a low fat or low carbohydrate diet. In these subjects key biomarkers, such as body weight, body fat, and plasma lipid profile, respond well to a diet balanced in fat and carbohydrate. For subjects with this genetic pattern who are interested in losing weight, a balanced diet restricted in calories has been found to promote weight loss and a decrease in body fat.

A low fat diet refers to a diet that provides between about 10% to less than about 40% of total calories from fat. A low fat diet refers to a diet that provides no more than about 35 percent (e.g., no more than about 19%, 21%, 23%, 22%, 24%, 26%, 28%, 33%, etc) of total calories from fat.

A low fat diet refers to a diet that provides no more than about 30 percent of total calories from fat. A low fat diet refers to a diet that provides no more than about 25 percent of total calories from fat. A low fat diet refers to a diet that provides no more than about 20 percent of total calories from fat.

A low fat diet refers to a diet that provides no more than about 15 percent of total calories from fat. A low fat diet refers to a diet that provides no more than about 10 percent of total calories from fat.

In some instances, a low fat diet refers to a diet that is between about 10 grams and about 60 grams of fat per day.

A low fat diet refers to a diet that is less than about 50 grams (*e.g.,* less than about 10, 25, 35, 45, etc) grams of fat per day.

A low fat diet refers to a diet that is less than about 40 grams of fat per day.

A low fat diet refers to a diet that is less than about 30 grams of fat per day. A low fat diet refers to a diet that is less than about 20 grams of fat per day.

Fats contain both saturated and unsaturated (monounsaturated and polyunsaturated) fatty acids. Reducing saturated fat to less than 10 percent of calories is a diet low in saturated fat.

Reducing saturated fat to less than 15 percent of calories is a diet low in saturated fat. Reducing saturated fat to less than 20 percent of calories is a diet low in saturated fat.

A low carbohydrate (CHO) diet refers to a diet that provides between about 20% to less than about 50% of total calories from carbohydrates. According to some embodiments, a low carbohydrate (CHO) diet refers to a diet that provides no more than about 50 percent (*e.g.,* no more than about 20%, 25%, 30%, 35%, 40%, 45%, etc) of total calories from carbohydrates. A low carbohydrate diet refers to a diet that provides no more than about 45 percent of total calories from carbohydrates. A low carbohydrate diet refers to a diet that provides no more than about 40 percent of total calories from carbohydrates. A low carbohydrate diet refers to a diet that provides no more than about 35 percent of total calories from carbohydrates.

A low carbohydrate diet refers to a diet that provides no more than about 30 percent of total calories from carbohydrates.

A low carbohydrate diet refers to a diet that provides no more than about 25 percent of total calories from carbohydrates.

A low carbohydrate diet refers to a diet that provides no more than about 20 percent of total calories from carbohydrates.

A low carbohydrate (CHO) diet may refer to a diet that restricts the amount of grams of carbohydrate in a diet such as a diet of from about 20 to about 250 grams of carbohydrates per day. Optionally, a low carbohydrate diet comprises no more than about 220 (*e.g.,* no more than about 40, 70, 90, 110, 130, 180, 210, etc) grams of carbohydrates per day. Optionally, a low carbohydrate diet comprises no more than about 200 grams of carbohydrates per day.

Optionally, a low carbohydrate diet comprises no more than about 180 grams of carbohydrates per day. Optionally, a low carbohydrate diet comprises no more than about 150 grams of carbohydrates per day.

Optionally, a low carbohydrate diet comprises no more than about 130 grams of carbohydrates per day. Optionally, a low carbohydrate diet comprises no more than about 100 grams of carbohydrates per day.

Optionally, a low carbohydrate diet comprises no more than about 75 grams of carbohydrates per day.

A calorie restricted diet or balanced diet refers to a diet that is restricts total calories consumed to below a subject's weight maintenance level (WML), regardless of any preference for a macronutrient. A balanced diet or calorie restricted diet seeks to reduce the overall caloric intake of a subject by, for example, reducing the total caloric intake of a subject to below that subject's WML without a particular focus on restricting the calories consumed from any particular macronutrient. Thus, a balanced diet may be expressed as a percentage of a subject's WML. For example, a balanced diet is a diet that comprises a total caloric intake of between about 50% to about 100% WML. For example, a balanced diet is a diet that comprises a total caloric intake of less than 100% (*e.g.,* less than about 99%, 97%, 95%, 90%, 85%, 80%, 75%, 70%, 65%, 60%, 55%) of WML. Within this framework, a balanced diet achieves a healthy or desired balance of macronutrients in the diet and may be: low fat; low saturated fat; low carbohydrate; low fat and low carbohydrate; or low saturated fat and low carbohydrate. For example, a diet may be a low fat, calorie restricted diet (where low fat has the meaning as provided hereinabove). A diet may be a low carbohydrate, calorie restricted diet (where low carbohydrate has the meaning as provided hereinabove). A diet may be a balanced, calorie restricted diet (*e.g.,* relative portions of macronutrients may vary where the total calories consumed is below the WML). For example, a low-carb diet (Carb: 45%, Protein: 20%, and Fat: 35%) comprises any of: Atkins diet, Glycemic Impact Diet, South Beach Diet, Sugar Busters Diet, and/or Zone diet.

Optionally, a low-fat diet (Carb: 65%, Protein: 15%, Fat:20%) comprises any of: Life Choice Diet (Ornish Diet), Pritikin Diet, and/or other heart healthy diets available in the market.

Optionally, a balanced diet (Carb: 55%, Protein: 20%, Fat: 25%) comprises any of: Best Life Diet, Mediterranean Diet, Sonoma Diet, Volumetrics Eating Diet, Weight Watchers Diet.

Other low carbohydrate, low fat, balanced diet and calorie restricted diets are well known in the art, thus can be recommended to a subject depending on the subject's metabolic genotype and predicted response to calorie restricted or other types of diet.

### EXERCISE CATEGORIES

Exercise categories are generally classified on the basis of how responsive a subject is to exercise given their metabolic genotype. For example, a subject may be responsive to light exercise, moderate exercise, heavy exercise, or very heavy exercise.

Subjects with a metabolic genotype that is responsive to exercise are able to effectively break down body fat in response to physical activity. They tend to respond to exercise with significant weight loss and are more likely to maintain that weight loss. Subjects fall into this category if they are responsive to light or moderate exercise.

Subjects with a metabolic genotype that is less responsive to exercise are less able to break down body fat for energy in response to exercise than those with the alternative genetic pattern. They tend to lose less weight and body fat than expected with moderate exercise. These subjects require more exercise to activate the breakdown of body fat for energy and weight loss. They must also maintain a consistent exercise program to keep the weight off.

Light activity generally refers to a subject that exercises (engages in an active workout or sports) 1-3 days per week. Moderate activity generally refers to a subject that exercises (engages in an active workout or sports) 3-5 days per week. High activity generally refers to a subject that exercises (engages in an active workout or sports) 6-7 days per week. Very high or extreme activity generally refers to a subject that exercises (engages in an active workout or sports) on average of more than once a day (*e.g.,* two times per day). Regular exercise refers to activity that is at least light exercise or at least moderate exercise.

More accurately, activity level may be expressed in terms of a percentage over BMR. For example, the multipliers of the Harris-Benedict or Katch-McArdle formulas may be used as a basis to define an activity level. Accordingly, light exercise refers to a recommended activity level designed to increase a subject's TDEE to about 125% of BMR (*i.e.,* about a 25% increase) to less than about 140% (*e.g.,* about 128%, 130%, 133%, 135%, 137.5%, etc) of BMR. Moderate exercise refers to a recommended activity level designed to increase a subject's TDEE to about 140% of BMR to less than about 160% (*e.g.,* about 142%, 145%, 150%, 155%, 158%, etc) of BMR. Heavy exercise refers to a recommended activity level designed to increase a subject's TDEE to about 160% of BMR to less than about 180% (*e.g.,* about 162%, 165%, 170%, 172.5%, 175%, 178%, etc) of BMR. Very heavy or extreme exercise refers to a recommended activity level designed to increase a subject's TDEE to about 180% of BMR to more than about 2*10% (e.g.,* about 182%, 185%, 190%, 195%, 200%, etc) of BMR.

Alternatively, according to some embodiments, a "normal exercise" routine comprises: 2.5 hours (150 minutes) of moderate-intensity activity per week (Moderate-intensity activities are defined as 3.0 to 5.9 METs), a "light exercise" routine comprises: less than 2.5 hours of moderate-intensity activity per week, and a "vigorous exercise" routine comprises: greater than 13 METs per week of vigorous intensity activities (Vigorous intensity activities are defined as 6 METs or greater). 1 MET is equal to 1 calorie/kg body mass/hour. The total kcal expended by a subject = MET value of activity x body weight in kg x time in hours.

Gain or loss of weight depends on a balance between calories consumed and calories expended. When the amount of calories consumed is greater than the number of calories expended, weight gain may occur. In contrast, if calories consumed is less than the number of calories expended, weight loss may occur. A subject's WML refers to the total caloric intake a subject needs to consume in order to maintain current body weight. A subject's WML may be determined or calculated using any method known in the art. WML is often expressed as total daily energy expenditure (TDEE) or estimated energy requirements (EER). While the meaning of TDEE and EER as used in the art may have technical distinctions reflecting the manner in which a subject's weight maintenance level is calculated, these terms may be used interchangeably in their general sense while maintaining their technical distinctions. WML may be calculated using any method used in the art (*e.g.,* TDEE or EER) to determine a subject's WML.

On average, for females in the U.S. the WML is between 2000-2100 calories per day. Males average a higher WML at 2700-2900 calories per day. A preferred method for calculating TDEE is by using the Harris-Benedict calculation or Katch-McArdle formula, which are well known to those of ordinary skill in the art. Briefly, the Harris-Benedict formula first determines and subject's basal metabolic rate (BMR), which is then adjusted base for activity level to give a subject's TDEE. For example, BMR for females may be calculated according to the following formula: BMR_{f}= 65.51 + (9.563 x kg) + (1.850 x cm) - (4.676 x age). BMR for males may be calculated according to the following formula: BMRₘ = 66.5 + (13.75 x kg) + (5.003 x cm) - (6.775 x age). The BMR is then adjusted by multiplying BMR by a multiplier assigned to a particular activity level. The table below provides examples of such multipliers. The result is a subject's TDEE.

**TABLE 4. Exercise Categories**

| TDEE | | |
|---|---|---|
| | Females | Males |
| Little or no exercise | BMR_{f} x 1.2 | BMRₘ x 1.2 |
| Light exercise | BMR_{f} x 1.375 | BMRₘ x 1.375 |
| Moderate exercise | BMR_{f} x 1.55 | BMRₘ x 1.55 |
| Heavy exercise | BMR_{f} x 1.725 | BMRₘ x 1.725 |
| Very heavy exercise | BMR_{f} x 1.9 | BMRₘ x 1.9 |

The Katch & McArdle formula is based on a subject's lean body mass (LBM). For example, BMR is calculated according to the following formula: BMR (men and women) = 370 + (21.6 X lean mass in kg). Since the Katch-McArdle formula accounts for LBM, this single formula applies equally to both men and women. TDEE is then determined using the activity multipliers as used in the Harris-Benedict calculation (in the table above).

### CLASSIFICATION

Generally, a subject's metabolic genotype will fall into a single nutrition category and a single exercise category. Thus, a subject will be classified into a nutrition category and exercise category based on their metabolic genotype. For example, a subject may be classified into one of the following six categories: 1) Responsive to Fat Restriction and Responsive to Exercise; 2) Responsive to Fat Restriction and Less Responsive to Exercise; 3) Responsive to Carbohydrate Restriction and Responsive to Exercise; 4) Responsive to Carbohydrate Restriction and Less Responsive to Exercise; 5) Balance of Fat and Carbohydrate and Responsive to Exercise; and 6) Balance of Fat and Carbohydrate and Less Responsive to Exercise.
1) Responsive to Fat Restriction and Responsive to Exercise: Subjects with this genetic pattern absorb more dietary fat into the body and have a slower metabolism. They have a greater tendency for weight gain. Clinical studies have shown these subjects have an easier time reaching a healthy body weight by decreasing total dietary fat. They may have greater success losing weight by following a reduced fat, reduced calorie diet. In addition, they benefit from replacing saturated fats with monounsaturated fats within a reduced calorie diet. Clinical studies have also shown these same dietary modifications improve the body's ability to metabolize sugars and fats.
   Subjects with this genetic pattern are able to effectively breakdown body fat in response to physical activity. They tend to respond to exercise with significant weight loss and are more likely to maintain that weight loss. Such subjects may benefit from any level of increased activity such as at least light exercise or at least moderate exercise.
2) Responsive to Fat Restriction and Less Responsive to Exercise -Subjects with this genetic pattern absorb more dietary fat into the body and have a slower metabolism. They have a greater tendency for weight gain. Clinical studies have shown these subjects have an easier time reaching a healthy body weight by decreasing total dietary fat. They may have greater success losing weight by following a reduced fat, reduced calorie diet. In addition, they benefit from replacing saturated fats with monounsaturated fats within a reduced calorie diet. Clinical studies have also shown these same dietary modifications improve the body's ability to metabolize sugars and fats.
   Subjects with this genetic pattern are less able to breakdown body fat for energy in response to exercise than those with the alternative genetic pattern. They tend to lose less weight and body fat than expected with moderate exercise. These subjects require more exercise to activate the breakdown of body fat for energy and weight loss. They must also maintain a consistent exercise program to keep the weight off.
3) Responsive to Carbohydrate Restriction and Responsive to Exercise - Subjects with this genetic pattern are more sensitive to weight gain from excessive carbohydrate intake. They may have greater success losing weight by reducing carbohydrates within a reduced calorie diet. Subjects with this genetic pattern are prone to obesity and have difficulty with blood sugar regulation if their daily carbohydrate intake exceeds 49% of total calories. Carbohydrate reduction has been shown to optimize blood sugar regulation and reduce risk of further weight gain. If they have high saturated and low monounsaturated fats in their diet, risk for weight gain and elevated blood sugar increases. While limiting total calories, these subjects may benefit from restricting total carbohydrate intake and shifting the fat composition of their diet to monounsaturated fats.
   Subjects with this genetic pattern are able to effectively breakdown body fat in response to physical activity. They tend to respond to exercise with significant weight loss and are more likely to maintain that weight loss.
4) Responsive to Carbohydrate Restriction and Less Responsive to Exercise - Subjects with this genetic pattern are more sensitive to weight gain from excessive carbohydrate intake. They may have greater success losing weight by reducing carbohydrates within a reduced calorie diet. Subjects with this genetic pattern are prone to obesity and have difficulty with blood sugar regulation if their daily carbohydrate intake exceeds 49% of total calories. Carbohydrate reduction has been shown to optimize blood sugar regulation and reduce risk of further weight gain. If they have high saturated and low monounsaturated fats in their diet, risk for weight gain and elevated blood sugar increases. While limiting total calories, these subjects may benefit from restricting total carbohydrate intake and shifting the fat composition of their diet to monounsaturated fats.
   Subjects with this genetic pattern are less able to breakdown body fat for energy in response to exercise than those with the alternative genetic pattern. They tend to lose less weight and body fat than expected with moderate exercise. These subjects require more exercise to activate the breakdown of body fat for energy and weight loss. They must also maintain a consistent exercise program to keep the weight off.
5) Balance of Fat and Carbohydrate and Responsive to Exercise - Subjects with this genetic pattern show no consistent need for a low fat or low carbohydrate diet. In these subjects key biomarkers, such as body weight, body fat, and plasma lipid profile, respond well to a diet balanced in fat and carbohydrate. For subjects with this genetic pattern who are interested in losing weight, a balanced diet restricted in calories has been found to promote weight loss and a decrease in body fat.
   Subjects with this genetic pattern are able to effectively breakdown body fat in response to physical activity. They tend to respond to exercise with significant weight loss and are more likely to maintain that weight loss.
6) Balance of Fat and Carbohydrate and Less Responsive to Exercise - Subjects with this genetic pattern show no consistent need for a low fat or low carbohydrate diet. In these subjects key biomarkers, such as body weight, body fat, and plasma lipid profile, respond well to a diet balanced in fat and carbohydrate. For subjects with this genetic pattern who are interested in losing weight, a balanced diet restricted in calories has been found to promote weight loss and a decrease in body fat.
   Subjects with this genetic pattern are less able to breakdown body fat for energy in response to exercise than those with the alternative genetic pattern. They tend to lose less weight and body fat than expected with moderate exercise. These subjects require more exercise to activate the breakdown of body fat for energy and weight loss. They must also maintain a consistent exercise program to keep the weight off.

In addition to the nutritional and exercise recommendations, the personalized therapeutic/dietary regimen may also include recommendation for dietary supplements, food supplements, or nutraceuticals. A "nutraceutical" is any functional food that provides an additional benefit other than its nutritional benefit. This category may include nutritional drinks, diet drinks (e.g., Slimfast™ and the like) as well as sports herbal and other fortified beverages.

### KITS

Kits are described for detecting metabolic genotype of a subject, comprising reagents (oligonucleotides, salts, enzymes, buffers, etc.) and instructions for using the kit.

Optionally, kits comprises a sample collection means, including, but not limited to a swab for collecting saliva, storage means for storing the collected sample, and for shipment. The kit further comprises a CD, or CD-ROM with instructions on how to collect sample, ship sample, and means to interpret genotypic information retrieved from the sample DNA, and translating the information into therapeutic/dietary or lifestyle recommendation. Genotype patterns can be stored, transmitted and displayed via computer networks and the internet. The therapeutic/dietary and lifestyle recommendations includes, but not limited to, those described herein.

### DETECTION OF ALLELES

Allelic patterns, polymorphism patterns, or haplotype patterns can be identified by detecting any of the component alleles using any of a variety of available techniques, including: 1) performing a hybridization reaction between a nucleic acid sample and a probe that is capable of hybridizing to the allele; 2) sequencing at least a portion of the allele; or 3) determining the electrophoretic mobility of the allele or fragments thereof (e.g., fragments generated by endonuclease digestion). The allele can optionally be subjected to an amplification step prior to performance of the detection step. Preferred amplification methods are selected from the group consisting of: the polymerase chain reaction (PCR), the ligase chain reaction (LCR), strand displacement amplification (SDA), cloning, and variations of the above (e.g. RT-PCR and allele specific amplification). Oligonucleotides necessary for amplification may be selected, for example, from within the metabolic gene loci, either flanking the marker of interest (as required for PCR amplification) or directly overlapping the marker (as in allele specific oligonucleotide (ASO) hybridization). In a particularly preferred embodiment, the sample is hybridized with a set of primers, which hybridize 5' and 3' in a sense or antisense sequence to the vascular disease associated allele, and is subjected to a PCR amplification.

An allele may also be detected indirectly, e.g. by analyzing the protein product encoded by the DNA. For example, where the marker in question results in the translation of a mutant protein, the protein can be detected by any of a variety of protein detection methods. Such methods include immunodetection and biochemical tests, such as size fractionation, where the protein has a change in apparent molecular weight either through truncation, elongation, altered folding or altered post-translational modifications.

A general guideline for designing primers for amplification of unique human chromosomal genomic sequences is that they possess a melting temperature of at least about 50° C, wherein an approximate melting temperature can be estimated using the formula Tₘₑₗₜ =[2X(# of A or T)+4X(# of G or C)].

Many methods are available for detecting specific alleles at human polymorphic loci. The preferred method for detecting a specific polymorphic allele will depend, in part, upon the molecular nature of the polymorphism. For example, the various allelic forms of the polymorphic locus may differ by a single base-pair of the DNA. Such single nucleotide polymorphisms (or SNPs) are major contributors to genetic variation, comprising some 80% of all known polymorphisms, and their density in the human genome is estimated to be on average 1 per 1,000 base pairs. SNPs are most frequently biallelic-occurring in only two different forms (although up to four different forms of an SNP, corresponding to the four different nucleotide bases occurring in DNA, are theoretically possible). Nevertheless, SNPs are mutationally more stable than other polymorphisms, making them suitable for association studies in which linkage disequilibrium between markers and an unknown variant is used to map disease-causing mutations. In addition, because SNPs typically have only two alleles, they can be genotyped by a simple plus/minus assay rather than a length measurement, making them more amenable to automation.

A variety of methods are available for detecting the presence of a particular single nucleotide polymorphic allele in a subject. Advancements in this field have provided accurate, easy, and inexpensive large-scale SNP genotyping. Most recently, for example, several new techniques have been described including dynamic allele-specific hybridization (DASH), microplate array diagonal gel electrophoresis (MADGE), pyrosequencing, oligonucleotide-specific ligation, the TaqMan system as well as various DNA "chip" technologies such as the Affymetrix SNP chips. These methods require amplification of the target genetic region, typically by PCR. Still other newly developed methods, based on the generation of small signal molecules by invasive cleavage followed by mass spectrometry or immobilized padlock probes and rolling-circle amplification, might eventually eliminate the need for PCR. Several of the methods known in the art for detecting specific single nucleotide polymorphisms are summarized below. The method of the present invention is understood to include all available methods.

Several methods have been developed to facilitate analysis of single nucleotide polymorphisms. The single base polymorphism can be detected by using a specialized exonuclease-resistant nucleotide, as disclosed, e.g., in Mundy, C. R. (U.S. Pat. No.4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

Alternatively, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA™ is described by Goelet, P. et al. (PCT Publication No. WO92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Publication No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A.-C., et al., Genomics 8:684-692 (1990); Kuppuswamy, M. N. et al., Proc. Natl. Acad. Sci. (U.S.A) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA™ in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

For mutations that produce premature termination of protein translation, the protein truncation test (PTT) offers an efficient diagnostic approach (Roest, et. al., (1993) Hum. Mol. Genet. 2:1719-2 1; van der Luijt, et. al., (1994) Genomics 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential in vitro transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

Any cell type or tissue may be utilized to obtain nucleic acid samples for use in the diagnostics described herein. Preferably, the DNA sample is obtained from a bodily fluid, e.g, blood, obtained by known techniques (e.g. venipuncture) or saliva. Alternatively, nucleic acid tests can be performed on dry samples (e.g. hair or skin). When using RNA or protein, the cells or tissues that may be utilized must express a metabolic gene of interest.

Diagnostic procedures may also be performed in situ directly upon tissue sections (fixed and/or frozen) of patient tissue obtained from biopsies or resections, such that no nucleic acid purification is necessary. Nucleic acid reagents may be used as probes and/or primers for such in situ procedures (see, for example, Nuovo, G. J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

A preferred detection method is allele specific hybridization using probes overlapping a region of at least one allele of a metabolic gene or haplotype and having about 5, 10, 20, 25, or 30 nucleotides around the mutation or polymorphic region. In a preferred embodiment of the invention, several probes capable of hybridizing specifically to other allelic variants of key metabolic genes are attached to a solid phase support, e.g., a "chip" (which can hold up to about 250,000 oligonucleotides). Oligonucleotides can be bound to a solid support by a variety of processes, including lithography. Mutation detection analysis using these chips comprising oligonucleotides, also termed "DNA probe arrays" is described e.g., in Cronin et al. (1996) Human Mutation 7:244. Optionally, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

These techniques may also comprise the step of amplifying the nucleic acid before analysis. Amplification techniques are known to those of skill in the art and include, but are not limited to cloning, polymerase chain reaction (PCR), polymerase chain reaction of specific alleles (ASA), ligase chain reaction (LCR), nested polymerase chain reaction, self sustained sequence replication (Guatelli, J. C. et al., 1990, Proc. Natl. Acad. Sci. USA 87:1874-1878), transcriptional amplification system (Kwoh, D. Y. et al., 1989, Proc. Natl. Acad. Sci. USA 86:1173-1177), and Q- Beta Replicase (Lizardi, P. M. et al., 1988, Bio/Technology 6:1197).

Amplification products may be assayed in a variety of ways, including size analysis, restriction digestion followed by size analysis, detecting specific tagged oligonucleotide primers in the reaction products, allele-specific oligonucleotide (ASO) hybridization, allele specific 5' exonuclease detection, sequencing, hybridization, and the like.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

For example, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize 5' and 3' to at least one allele of a metabolic gene or haplotype under conditions such that hybridization and amplification of the allele occurs, and (iv) detecting the amplification product. These detection schemes are especially useful for the detection of nucleic acid molecules if such molecules are present in very low numbers.

Preferably, in the described assay, the allele of a metabolic gene or haplotype is identified by alterations in restriction enzyme cleavage patterns. For example, sample and control DNA is isolated, amplified (optionally), digested with one or more restriction endonucleases, and fragment length sizes are determined by gel electrophoresis.

Any of a variety- of sequencing reactions known in the art can be used to directly sequence the allele. Exemplary sequencing reactions include those based on techniques developed by Maxim and Gilbert ((1977) Proc. Natl Acad Sci USA 74:560) or Sanger (Sanger et al (1977) Proc. Nat. Acad. Sci USA 74:5463). It is also contemplated that any of a variety of automated sequencing procedures may be utilized when performing the subject assays (see, for example Biotechniques (1995) 19:448), including sequencing by mass spectrometry (see, for example PCT publication WO 94/16101; Cohen et al. (1996) Adv Chromatogr 36:127-162; and Griffin et al. (1993) Appl Biochem Biotechnol 38:147-159). It will be evident to one of skill in the art that, for certain embodiments, the occurrence of only one, two or three of the nucleic acid bases need be determined in the sequencing reaction. For instance, A-track or the like, e.g., where only one nucleic acid is detected, can be carried out.

Optionally, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al. (1985) Science 230:1242). In general, the art technique of "mismatch cleavage" starts by providing heteroduplexes formed by hybridizing (labeled) RNA or DNA containing the wild-type allele with the sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al (1988) Proc. Natl Acad Sci USA 85:4397; and Saleeba et al (1992) Methods Enzymol. 217:286-295. Preferably, the control DNA or RNA can be labeled for detection.

The mismatch cleavage reaction may employ one or more proteins that recognize mismatched base pairs in double-stranded DNA (so called "DNA mismatch repair" enzymes). For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). For example, a probe based on an allele of a metabolic gene locus haplotype is hybridized to a CDNA or other DNA product from a test cell(s). The duplex is treated with a DNA mismatch repair enzyme, and the cleavage products, if any, can be detected from electrophoresis protocols or the like. See, for example, U.S. Pat. No. 5,459,039.

Alternatively, alterations in electrophoretic mobility will be used to identify a metabolic gene locus allele. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control metabolif locus alleles are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. Preferably, the subject method utilizes heteroduplex analysis to separate double stranded heteroduplex molecules on the basis of changes in electrophoretic mobility (Keen et al. (1991) Trends Genet 7:5).

Alternatively, the movement of alleles in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. Alternatively, a temperature gradient is used in place of a denaturing agent gradient to identify differences in the mobility of control and sample DNA (Rosenbaum and Reissner (1987) Biophys Chem 265:12753).

Examples of other techniques for detecting alleles include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al. (1986) Nature 324:163); Saiki et al (1989) Proc. Natl Acad. Sci USA 86:6230). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used. Oligonucleotides used as primers for specific amplification may carry the mutation or polymorphic region of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 1 1:238). In addition it may be desirable to introduce a novel restriction site in the region of the mutation to create cleavage-based detection (Gasparini et al (1992) Mol. Cell Probes 6:1). It is anticipated that in certain instances amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

Identification of the allelic variant may be carried out using an oligonucleotide ligation assay (OLA), as described, e.g., in U.S. Pat. No. 4,998,617 and in Landegren, U. et al. ((1988) Science 241:1077-1080). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al. (1990) Proc. Natl. Acad. Sci. USA 87:8923-27). In this method, PCR is used to achieve the exponential amplification of target DNA, which is then detected using OLA.

Several techniques based on this OLA method have been developed and can be used to detect alleles of a metabolic gene locus haplotype. For example, U.S. Pat. No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996) Nucleic Acids Res 24: 3728), OLA combined with PCR permits typing of two alleles in a single microtiter well. By marking each of the allele-specific primers with a unique hapten, i.e. digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

We also disclose kits for performing the above-described assays. The kits may include a means for determining a subject's genotype with respect to one or more metabolic gene. The kit may also contain a nucleic acid sample collection means. The kit may also contain a control sample either positive or negative or a standard and/or an algorithmic device for assessing the results and additional reagents and components including: DNA amplification reagents, DNA polymerase, nucleic acid amplification reagents, restrictive enzymes, buffers, a nucleic acid sampling device, DNA purification device, deoxynucleotides, oligonucleotides (e.g. probes and primers) etc.

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

As described above, the control may be a positive or negative control. Further, the control sample may contain the positive (or negative) products of the allele detection technique employed. For example, where the allele detection technique is PCR amplification, followed by size fractionation, the control sample may comprise DNA fragments of the appropriate size. Likewise, where the allele detection technique involves detection of a mutated protein, the control sample may comprise a sample of mutated protein. However, it is preferred that the control sample comprises the material to be tested. For example, the controls may be a sample of genomic DNA or a cloned portion of a metabolic gene. Preferably, however, the control sample is a highly purified sample of genomic DNA where the sample to be tested is genomic DNA.

The oligonucleotides present in said kit may be used for amplification of the region of interest or for direct allele specific oligonucleotide (ASO) hybridization to the markers in question. Thus, the oligonucleotides may either flank the marker of interest (as required for PCR amplification) or directly overlap the marker (as in ASO hybridization).

Information obtained using the assays and kits described herein (alone or in conjunction with information on another genetic defect or environmental factor, which contributes to osteoarthritis) is useful for determining whether a non-symptomatic subject has or is likely to develop the particular disease or condition. In addition, the information can allow a more customized approach to preventing the onset or progression of the disease or condition. For example, this information can enable a clinician to more effectively prescribe a therapy that will address the molecular basis of the disease or condition.

The kit may, optionally, also include DNA sampling means. DNA sampling means are well known to one of skill in the art and can include, but not be limited to substrates, such as filter papers, the AmpliCard™ (University of Sheffield, Sheffield, England S10 2JF; Tarlow, J W, et al., J. of Invest. Dermatol. 103:387-389 (1994)) and the like; DNA purification reagents such as Nucleon™ kits, lysis buffers, proteinase solutions and the like; PCR reagents, such as 10X reaction buffers, thernostable polymerase, dNTPs, and the like; and allele detection means such as the Hinfl restriction enzyme, allele specific oligonucleotides, degenerate oligonucleotide primers for nested PCR from dried blood.

We also disclose kits for detecting a predisposition for responsiveness to certain diets and/or activity levels. This kit may contain one or more oligonucleotides, including 5' and 3' oligonucleotides that hybridize 5' and 3' to at least one allele of a metabolic gene locus or haplotype. PCR amplification oligonucleotides should hybridize between 25 and 2500 base pairs apart, preferably between about 100 and about 500 bases apart, in order to produce a PCR product of convenient size for subsequent analysis.

**TABLE 5: Particularly preferred primers for use in the method of the invention included are listed.**

| **Gene** | **SNP** | **PCR primer** | **Position** | **Sequence** | **Position** | **PCR product size (bp)** |
|---|---|---|---|---|---|---|
| **FABP22** | **rs1799883** | FA_F1 | 5' | TGTTCTTGTGCAAAGGCAA TGCTACCG | 3' | 311 |
| | | FA_R1 | 5' | TCTTACCCTGAGTTCAGTTC CGTCTGC | 3' | |
| **ADRB2** | **rs1042713** | A1_F1 | 5' | GCCCCTAGCACCCGACAAG CTGAGTGT | 3' | 422 |
| | **rs1042714** | A2_R1 | 5' | CCAGGCCCATGACCAGATC AGCACAG | 3' | |
| | | | | 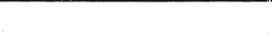 | | |
| **ADRB23** | **rs4994** | A3_F2 | 5' | AAGCGTCGCTACTCCTCCC CCAAGAGC | 3' | 569 |
| | | A3_R2 | 5' | GTCACACACAGCACGTCCA CCGAGGTC | 3' | |
| **PPARG** | **rs1801282** | PP_F1 | 5' | TGCCAGCCAATTCAAGCCC AGTCCTTT | 3' | 367 |
| | | PP_R1 | 5' | ACACAACCTGGAAGACAA ACTACAAGAGCAA | 3' | |
| | | | | | | |

| **Gene** | | **SBE primer** | | **Sequence** | | |
|---|---|---|---|---|---|---|
| **FABP2** | **rs1799883** | SBE_FA_F1 | 5' | GAAGGAAATAAATTCACA GTCAAAGAATCAAGC | 3' | |
| | | | | | | |
| **ADRB2** | **rs1042713** | SBE_A1_F2 | 5' | AACGGCAGCGCCTTCTTGC TGGCACCCAAT | 3' | |
| | | | | | | |
| | **rs1042714** | SBE_A2 F1 | 5' | AGCCATGCGCCGGACCACG ACGTCACGCAG | 3' | |
| | | | | | | |
| **ADRB3** | **rs4994** | SBE_A3_F3 | 5' | GGGAGGCAACCTGCTGGTC ATCGTGGCCATCGCC | 3' | |
| | | | | | | |
| **PPARG** | **rs1801282** | SBE_PP_R1 | 5' | GACAGTGTATCAGTGAAGG AATCGCTTTCTG | 3' | |
| | | | | | | |
| PCR= Polymerase Chain Reaction | | | | | | |
| SBE= Single Base Extension | | | | | | |

The design of additional oligonucleotides for use in the amplification and detection of metabolic gene polymorphic alleles by the method of the invention is facilitated by the availability of both updated sequence information from human chromosome 4q28-q31 --which contains the human FABP2 locus, and updated human polymorphism information available for this locus. Suitable primers for the detection of a human polymorphism in metabolic genes can be readily designed using this sequence information and standard techniques known in the art for the design and optimization of primers sequences. Optimal design of such primer sequences can be achieved, for example, by the use of commercially available primer selection programs such as Primer 2.1, Primer 3 or GeneFisher (See also, Nicklin M. H. J., Weith A. Duff G. W., "A Physical Map of the Region Encompassing the Human Interleukin-1α, interleukin-1β, and Interleukin-1 Receptor Antagonist Genes" Genomics 19: 382 (1995); Nothwang H. G., et al. "Molecular Cloning of the Interleukin-1 gene Cluster: Construction of an Integrated YAC/PAC Contig and a partial transcriptional Map in the Region of Chromosome 2q13" Genomics 41: 370 (1997); Clark, et al. (1986) Nucl. Acids. Res., 14:7897-7914 [published erratum appears in Nucleic Acids Res., 15:868 (1987) and the Genome Database (GDB) project).

We also describe kits for performing the above-described assays. The kits may include a means for determining a subject's genotype with respect to one or more metabolic gene. The kit may also contain a nucleic acid sample collection means. The kit may also contain a control sample either positive or negative or a standard and/or an algorithmic device for assessing the results and additional reagents and components including: DNA amplification reagents, DNA polymerase, nucleic acid amplification reagents, restrictive enzymes, buffers, a nucleic acid sampling device, DNA purification device, deoxynucleotides, oligonucleotides (e.g. probes and primers) etc.

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

As described above, the control may be a positive or negative control. Further, the control sample may contain the positive (or negative) products of the allele detection technique employed. For example, where the allele detection technique is PCR amplification, followed by size fractionation, the control sample may comprise DNA fragments of the appropriate size. Likewise, where the allele detection technique involves detection of a mutated protein, the control sample may comprise a sample of mutated protein. However, it is preferred that the control sample comprises the material to be tested. For example, the controls may be a sample of genomic DNA or a cloned portion of a metabolic gene. Preferably, however, the control sample is a highly purified sample of genomic DNA where the sample to be tested is genomic DNA.

The oligonucleotides present in said kit may be used for amplification of the region of interest or for direct allele specific oligonucleotide (ASO) hybridization to the markers in question. Thus, the oligonucleotides may either flank the marker of interest (as required for PCR amplification) or directly overlap the marker (as in ASO hybridization).

Information obtained using the assays and kits described herein (alone or in conjunction with information on another genetic defect or environmental factor, which contributes to osteoarthritis) is useful for determining whether a non-symptomatic subject has or is likely to develop the particular disease or condition. In addition, the information can allow a more customized approach to preventing the onset or progression of the disease or condition. For example, this information can enable a clinician to more effectively prescribe a therapy that will address the molecular basis of the disease or condition.

The kit may, optionally, also include DNA sampling means. DNA sampling means are well known to one of skill in the art and can include, but not be limited to substrates, such as filter papers, the AmpliCard™ (University of Sheffield, Sheffield, England S10 2JF; Tarlow, J W, et al., J. of Invest. Dermatol. 103:387-389 (1994)) and the like; DNA purification reagents such as Nucleon™ kits, lysis buffers, proteinase solutions and the like; PCR reagents, such as 10X reaction buffers, thernostable polymerase, dNTPs, and the like; and allele detection means such as the Hinfl restriction enzyme, allele specific oligonucleotides, degenerate oligonucleotide primers for nested PCR from dried blood.

### Definitions

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

In the case of conflict, the present specification, including definitions, will control. In addition, the materials, methods, and examples are illustrative only and not intended to be limiting. Other features and advantages of the invention will be apparent from the following detailed description and claims.

For the purposes of promoting an understanding of the embodiments described herein, reference will be made to preferred embodiments and specific language will be used to describe the same. The terminology used herein is for the purpose of describing particular embodiments only, and is not intended to limit the scope of the present invention. As used throughout this disclosure, the singular forms "a," "an," and "the" include plural reference unless the context clearly dictates otherwise. Thus, for example, a reference to "a composition" includes a plurality of such compositions, as well as a single composition, and a reference to "a therapeutic agent" is a reference to one or more therapeutic and/or pharmaceutical agents and equivalents thereof known to those skilled in the art, and so forth.

The term "allele" refers to the different sequence variants found at different polymorphic regions. The sequence variants may be single or multiple base changes, including without limitation insertions, deletions, or substitutions, or may be a variable number of sequence repeats.

The term "allelic pattern" refers to the identity of an allele or alleles at one or more polymorphic regions. For example, an allelic pattern may consist of a single allele at a polymorphic site, as for PPARG (rs1801282) allele 1. Alternatively, an allelic pattern may consist of either a homozygous or heterozygous state at a single polymorphic site. For example, PPARG (rs1801282) allele 2.2 is an allelic pattern in which there are two copies of the second allele and corresponds to the homozygous PPARG (rs1801282) allele 2 state. Alternatively, an allelic pattern may consist of the identity of alleles at more than one polymorphic site.

The terms "control" or "control sample" refer to any sample appropriate to the detection technique employed. The control sample may contain the products of the allele detection technique employed or the material to be tested. Further, the controls may be positive or negative controls. By way of example, where the allele detection technique is PCR amplification, followed by size fractionation, the control sample may comprise DNA fragments of an appropriate size. Likewise, where the allele detection technique involves detection of a mutated protein, the control sample may comprise a sample of a mutant protein. However, it is preferred that the control sample comprises the material to be tested. For example, the controls may be a sample of genomic DNA or a cloned portion containing one or more metabolic genes. However, where the sample to be tested is genomic DNA, the control sample is preferably a highly purified sample of genomic DNA.

The phrases "disruption of the gene" and "targeted disruption" or any similar phrase refers to the site specific interruption of a native DNA sequence so as to prevent expression of that gene in the cell as compared to the wild-type copy of the gene. The interruption may be caused by deletions, insertions or modifications to the gene, or any combination thereof.

The term "haplotype" as used herein is intended to refer to a set of alleles that are inherited together as a group (are in linkage disequilibrium) at statistically significant levels (P_{corr} <0.05). As used herein, the phrase "metabolic haplotype" refers to a haplotype of metabolic gene loci.

"Increased risk" refers to a statistically higher frequency of occurrence of the disease or condition in a subject carrying a particular polymorphic allele in comparison to the frequency of occurrence of the disease or condition in a member of a population that does not carry the particular polymorphic allele.

The term "isolated" as used herein with respect to nucleic acids, such as DNA or RNA, refers to molecules separated from other DNAs, or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

"Linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage disequilibrium". The cause of linkage disequilibrium is often unclear. It can be due to selection for certain allele combinations or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in the specific chromosomal region. When referring to allelic patterns that are comprised of more than one allele, a first allelic pattern is in linkage disequilibrium with a second allelic pattern if all the alleles that comprise the first allelic pattern are in linkage disequilibrium with at least one of the alleles of the second allelic pattern.

The term "marker" refers to a sequence in the genome that is known to vary among subjects.

A "mutated gene" or "mutation" or "functional mutation" refers to an allelic form of a gene, which is capable of altering the phenotype of a subject having the mutated gene relative to a subject which does not have the mutated gene. The altered phenotype caused by a mutation can be corrected or compensated for by certain agents. If a subject must be homozygous for this mutation to have an altered phenotype, the mutation is said to be recessive. If one copy of the mutated gene is sufficient to alter the phenotype of the subject, the mutation is said to be dominant. If a subject has one copy of the mutated gene and has a phenotype that is intermediate between that of a homozygous and that of a heterozygous subject (for that gene), the mutation is said to be co-dominant.

As used herein, the term "nucleic acid" refers to polynucleotides or oligonucleotides such as deoxyribonucleic acid (DNA), and, where appropriate, ribonucleic acid (RNA). The term should also be understood to include, as equivalents, analogs of either RNA or DNA made from nucleotide analogs (e.g. peptide nucleic acids) and as applicable to the embodiment being described, single (sense or antisense) and double-stranded polynucleotides.

The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A specific genetic sequence at a polymorphic region of a gene is an allele. A polymorphic region can be a single nucleotide, the identity of which differs in different alleles. A polymorphic region can also be several nucleotides long.

The term "propensity to disease," also "predisposition" or "susceptibility" to disease or any similar phrase, means that certain alleles are hereby discovered to be associated with or predictive of a subject's incidence of developing a particular disease (e.g. a vascular disease). The alleles are thus over-represented in frequency in subjects with disease as compared to healthy subjects. Thus, these alleles can be used to predict disease even in pre-symptomatic or pre-diseased subjects.

As used herein, the term "specifically hybridizes" or "specifically detects" refers to the ability of a nucleic acid molecule to hybridize to at least approximately 6 consecutive nucleotides of a sample nucleic acid.

"Transcriptional regulatory sequence" is a generic term used throughout the specification to refer to DNA sequences, such as initiation signals, enhancers, and promoters, which induce or control transcription of protein coding sequences with which they are operably linked.

The term "vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

The following examples are illustrative, but not limiting, of the methods and compositions of the present invention.

### EXAMPLE 1

A weight management test has been developed from a comprehensive review of clinical studies identifying correlations between genes and variations in weight management-related metabolism; establishing acceptance criteria to identify which genetic variations affect metabolic pathways in ways that are potentially modifiable by changes in diet and lifestyle; determining which genotypes have been shown to increase risk and that suggest a risk that may be modifiable by diet and/or lifestyle intervention; and compiling evidence to support the test configuration chosen, test result interpretations, dietary/lifestyle interventions, and benefit/risk analysis.

The gene/polymorphism selection criteria required evidence that: the polymorphism has a significant association with a weight management phenotype (e.g., weight, body fat, body mass index) as seen in evidence from three or more independent, similar studies that showed the same genotype association; the gene has a biologically plausible role in weight management; the polymorphism is associated with a functional impact either at the molecular genetic level or as determined by measurement of biomarkers known to influence weight and/or health outcomes; and an intervention response (e.g., diet or exercise) has been shown to differ by genotype, as seen in evidence from two or more independent, similar studies of polymorphism genotype leading to a specific recommendation category.

### Scientific Rational For The Test Panel

The scientific rationale for this test is based on an extensive review of the scientific literature available through April 2007. The published evidence was evaluated against a prospectively articulated set of acceptance criteria. This evidence was assembled in the hierarchy of gene > polymorphism > composite genotype to define and justify the test result interpretations for the panel.

The evaluation process included:
1. Establishing candidate genes by identifying significant involvement in metabolic pathways related to weight homeostasis.
2. Establishing acceptance criteria to decide which genetic variations affect metabolic pathways in ways that are potentially modifiable by changes in diet and exercise patterns. These included evidence that:
   a) The polymorphism has a significant association with a relevant phenotype (weight, body fat, or body mass index) as demonstrated by three or more independent studies that showed the same genotype-phenotype association.
   b) The gene has a biologically plausible role in weight management.
   c) The polymorphism is associated with a functional impact either at the DNA level or as determined by measurement of biomarkers known to be associated with physiological pathways that affect weight homeostasis.
   d) The response of subjects to interventions such as diet or exercise can be stratified by genotype. Such evidence must be presented in at least two independent.
3. Conducting a comprehensive search of the scientific literature to evaluate the impact of genetic variations on: a) metabolic mechanisms; b) obesity/weight management and health outcome associations, and c) responses to intervention as measured by change in weight or adiposity or biomarker changes.
4. Determining which genotypes have been shown to predispose a subject to weight gain and that the gain may be modifiable by a particular dietary or exercise strategy.
5. Compiling evidence to support the test configuration chosen, test result interpretations, dietary/lifestyle interventions, and benefit/risk analysis.

The following genes have met the criteria outlined above. They have been selected for their impact on various pathways that influence body weight and have been associated with elevated risk for obesity. They have also been selected because they may be used to differentiate response to weight management interventions by genotype. They are: Fatty Acid Binding Protein 2 (FABP2); Peroxisome Proliferator-Activated Receptor-Gamma (PPARG); Beta-2 Adrenergic Receptor (ADRB2); and Beta-3 Adrenergic Receptor (ADRB3).

### Rational for Composite Genotypes

After identification of the gene/polymorphisms that met or exceeded the prospectively developed criteria for inclusion in the test panel, combinations were analyzed to determine if the composite genotypes encountered for all five polymorphisms could be partitioned into distinct categories that would support specific interpretations. Results were divided into three categories based on evidence of response to dietary macronutrients (Responsive to Fat Restriction, Responsive to Carbohydrate Restriction, and Balance of Fat and Carbohydrate). They also were partitioned into two separate categories based on evidence of response to exercise (Responsive to Exercise and Less Responsive to Exercise). The resulting three by two (six cell) matrix of categories or genotype patterns is shown in Table 7.

### Responsive to Fat Restricted Diet

This category is composed of persons with the composite genotypes: FABP2 Ala54Thr and PPARG Pro12Ala. Those with the PPARG 12Pro/Pro genotype who are also carriers of the FABP2 Thr54 allele are also in this category. These subjects demonstrate difficulties in weight management without restricting specific fat intakes. The FABP2 Thr54 variant has a two-fold greater binding affinity for long-chain fatty (1) acids and enhanced fat absorption and/or processing of dietary fatty acids by the intestine (2). The Thr54 variant increases absorption and/or processing of dietary fatty acids by the intestine. PPARG plays a key role in the formation of fat cells (fat storage) and in lipid metabolism (fat mobilization). PPARG is a receptor located in the nucleus of fat cells. When activated by dietary fat, the PPARG receptor binds to specific DNA sequences which then "turns on" certain genes that promote storage of dietary fat in fat cells. In humans, enhanced PPARG activity is associated with increased adiposity. The Ala12 variant is associated with a reduced PPARG activity (43, 44). Persons who are 12Pro/ Pro are likely to be more responsive to the amount of dietary fat than are the 12 Ala carriers. Carriers of the Ala12 variant have greater metabolic flexibility in the storage and mobilization of fat in response to intervention. Thus, subjects who are 12Pro/Pro are more efficient at accumulating fat from the diet. Compared to Ala12 carriers, those with the 12Pro/ Pro genotype have enhanced binding of PPARG to DNA, which leads to more efficient activation of the receptor and promotes fat storage.

### Responsive to Carbohydrate Restriction

This category includes those persons with either one of two different genetic combinations: PPARG Pro12Ala and ADRB2 Gln27Glu. Persons who have the PPARG 12Ala/* genotype (Ala allele carriers) and/or carry the ADRB2 Glu27 allele have difficulties in weight management unless they restrict dietary intake of carbohydrate. In two separate studies, each focused on only one of the two gene/SNPs, investigators found a decreased tendency to weight gain/obesity in subjects carrying the variant allele when their carbohydrate intake was restricted to less than 50% of total calories when compared to those with the same genotypes whose intake was above 50% (30, 38). This suggests that each of these variations shows differences in risk for obesity under carbohydrate restriction. In addition, one of these studies demonstrated a reduced risk of insulin resistance in subjects carrying the variant allele when their carbohydrate intake was less than 50% of total calories (30). Results from intervention studies with Ala12 carriers indicate they have greater weight loss (18) and greater improvements in insulin sensitivity in response to a low-calorie diet (19) and exercise training (45-47) than do non-carriers. These results may be explained by the reduced PPARG activity associated with the Ala12 variant, which results in less efficient stimulation of PPARG target genes, causing less adiposity (reduced capacity to store fat) and in turn greater insulin sensitivity. It is appropriate to recommend a carbohydrate restricted diet to carriers of Ala12 or Glu27 alleles because being a carrier of either increases the risk of obesity on a high carbohydrate diet, and these genotypes are associated with improvements in insulin sensitivity in conjunction with diet/exercise interventions.

The results of intervention studies that use change in weight and insulin sensitivity are strong for PPARG 12Ala/* and for ADRB2 27Glu/* (18,30,38,45-47). However, no studies evaluated the effects of both polymorphisms in one population. Thus, it is more appropriate to include PPARG 12Ala/* "and/or" ADRB2 27Glu/* genotype subjects into this pattern than to require the combination of both SNP genotypes.

The only contradiction among the 5 SNP genotype patterns is when subjects carrying the ADRB2 Glu27 allele also have the combination of PPARG 12Pro/Pro and FABP2 54Thr/*, which would qualify them for the "Responsive to Fat Restriction" pattern. The test assigns such subjects to the "Responsive to Fat Restriction" pattern, because the preponderance of scientific evidence for the gene-diet interaction of PPARG and FABP2 polymorphisms on body weight and/or body fat related phenotypes (1,2,9,10,16,18) is greater than that found for the gene-diet interactions of ADRB2 for body responses to carbohydrate modulation (21,30,31).

Multiple studies have demonstrated that subjects who carry the FABP2 Thr54 allele are at risk of metabolic syndrome (48-50). Others have demonstrated an improvement in glucose metabolism-related risk factors (insulin, blood sugar, triglycerides) through reduction of saturated fat intake (10,11,12). The intervention research that focused on the type of fat in the diet also included, in most cases, a moderate amount of dietary carbohydrate. Other research that does not directly link to FABP2 genotype demonstrates an improvement in insulin levels and blood glucose control by modulating carbohydrate intake (51-53). Rather than focusing on reducing the fat in their diet; subjects with the combined PPARG 12/Ala/* and FABP2 54Thr/* genotype would likely benefit more from reducing their carbohydrate intake.

### Less Responsive to Exercise

Persons who have a specific genotype in either the ADRB3 gene or the ADRB2 gene have a genetic predisposition that tends to make them less responsive to exercise as a strategy to control weight. Both of these polymorphisms play a key role in the mobilization of fat from adipose tissue (lipolysis) by mediating the response to catecholamines. The ADRB2 Gly16 variant, (even when combined with the Glu27 variant during in vitro studies), is associated with a lower adrenergic receptor responsiveness (21). These two polymorphisms are in close linkage disequilibrium. Thus, testing for the Gly16 variant also identifies most subjects carrying the Glu27 variant, which has been associated with same predisposition. The ADRB3 Arg64 variant is associated with reduced receptor function and reduced lipolysis. During exercise, carriers of the variant are likely to exhibit a reduced lipolysis and thus a reduced capacity to burn fat, which would result in less weight loss in response to exercise. Multiple intervention studies have consistently shown that persons with the Arg64 variant have more difficulty losing weight in response to diet/exercise than do non-carriers. Carriers of the Gly16 variant of ADRB2 are less likely than non-carriers to lose weight through exercise (23) or a combination of diet and exercise (28). Considering that both adrenergic receptors influence response to catecholamines during exercise, and that both ADRB2 Gly16 and ADRB3 Arg64 have reduced receptor function, subjects with either of these polymorphisms should be included in the less exercise responsive composite pattern.

Results were divided into three separate categories based on evidence of response to dietary macronutrients, and into two separate categories based on evidence of response to exercise. The resulting three by two matrix of categories or genotype patterns is shown below (Table 6).

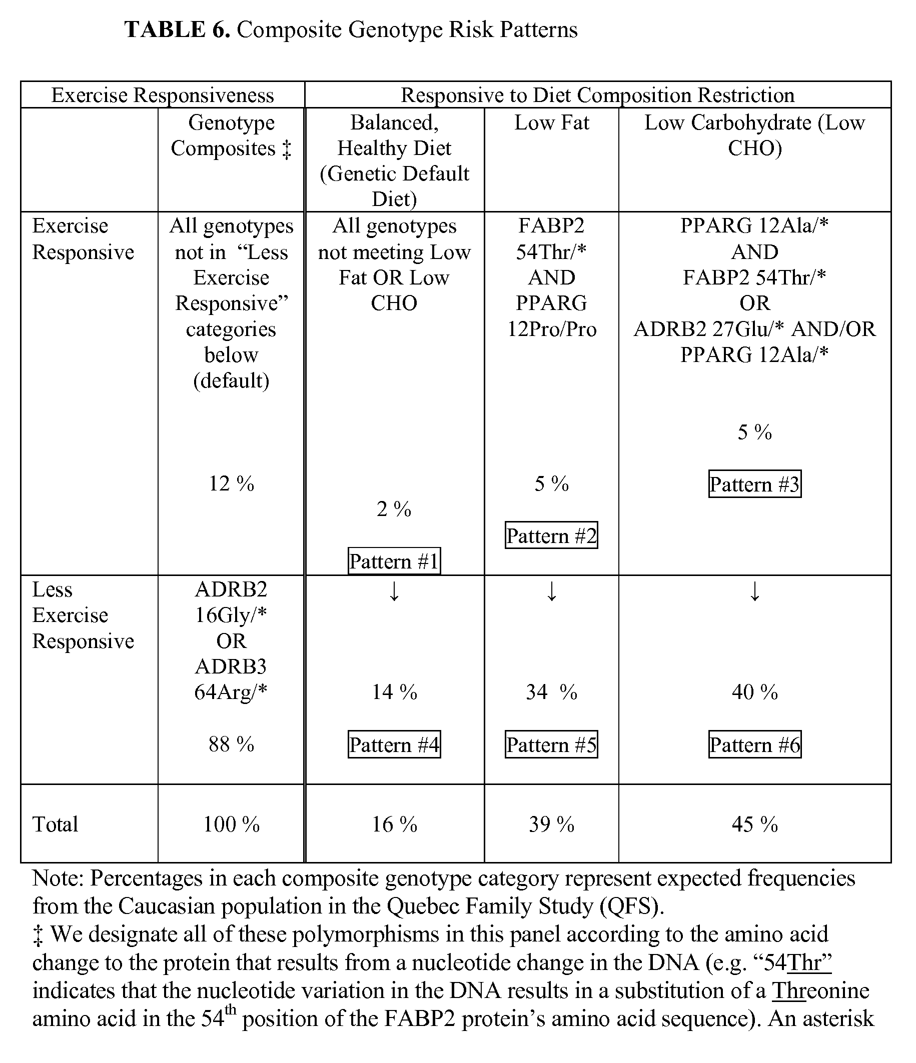

Composite Genotype Pattern #1 -Responsive to a Balance of Fat and Carbohydrate, Responsive to Exercise: Subjects with a combined genotype of FABP2 rs1799883, 1.1 or G/G (54Ala/Ala), PPARG rs1801282, 1.1 or C/C (12Pro/Pro), and ADRB2 rs1042714, 1.1 or C/C (27Gln/Gln), and ADRB2 rs1042713 2.2 or A/A (16Arg/Arg), and ADRB3 rs4994 1.1 or T/T (64Trp/Trp). This category includes subject genotypes known to be responsive with weight differences from low fat or low carbohydrate, calorie-restricted diets. From the variants tested in this panel, these subjects show no consistent genetic tendency towards impaired response, isolated to either fats or carbohydrates in their diet. They show a normal energy metabolism response to regular exercise for achieving their weight management goals. This composite genotype is present in 2% of the Caucasian population.

Composite Genotype Pattern #2 - Responsive to Fat Restriction, Responsive to Exercise: Subjects with a combined genotype of FABP2 rs1799883, 2.2 or 1.2 (A/A or G/A) (54Thr/*) and PPARG rs1801282, 1.1 or C/C (12Pro/Pro), and either ADRB2 rs1042714, 1.2 or 2.2 (C/G or G/G) (27Glu*) or ADRB2 rs1042714, 1.1 (C/C) (27Gln/Gln), in combination with ADRB2 rs1042713, 2.2 (A/A) (16Arg/Arg) and ADRB3 rs4994, 1.1 (T/T) (64Trp/Trp). These subjects absorb more of their dietary fat and tend to store it in fat cells, rather than mobilize it during metabolism. They show a normal energy metabolism response to regular exercise for achieving their weight management goals. This composite genotype is expected in about 5% of the Caucasian population.

Composite Genotype Pattern #3 - Responsive to Carbohydrate Restriction, Responsive to Exercise: Subjects whose genotypes include PPARG rs1801282 (12Ala/*) 1.2 or 2.2 (C/G or G/G) and/or ADRB2 rs1042714 (27Glu/*) 1.2 or 2.2 (C/G or G/G), as well as subjects with a combined genotype of PPARG rs1801282 (12Ala/*) 1.2 or 2.2 (C/G or G/G) and FABP2 rs1799883 (54Thr/*) 2.2 or 1.2 (A/A or G/A). All of the above qualifying genotypes will be in combination with ADRB2 rs 1042713 (16 Arg/Arg) 2.2 (A/A) and ADRB3 rs4994 (64 Trp/Trp) 1.1 (T/T) to meet the responsive to exercise category requirement. These subjects tend to gain or retain weight from high dietary carbohydrate intake, and show signs of impaired glucose and insulin metabolism. They show a normal energy metabolism response to regular exercise for achieving their weight management goals. This composite genotype is expected in about 5 % of the Caucasian population.

Composite Genotype Patterns #4 - Responsive to a Balance of Fat and Carbohydrate, Less Responsive to Exercise: Subjects with a combined genotype of FABP2 rs 1799883 (54Ala/Ala) 1.1 (G/G) and PPARG rs1801282 (12Pro/Pro) 1.1 (C/C) and ADRB2 rs1042713 (16Gly*) 1.2 or 1.1 (G/G or G/A) or ADRB3 rs4994 (64Arg*) 1.2 or 2.2 (C/T or C/C). This category includes subject genotypes known to be responsive with weight differences from low fat or low carbohydrate, calorie-restricted diets. From the variants tested in this panel, these subjects show no consistent genetic tendency towards impaired response, isolated to either fats or carbohydrates in their diet. They tend to have impaired energy metabolism and to be less responsive to regular exercise for achieving their weight management goals. This composite genotype is present in 14% of the Caucasian population.

Composite Genotype Pattern #5 - Responsive to Fat Restriction, Less Responsive to Exercise: Subjects with a combined genotype of FABP2 rs1799883 (54Thr/*) 2.2 or 2.1 (A/A or A/G) and PPARG rs1801282 (12Pro/Pro) 1.1 (C/C), and either ADRB2 rs1042714 (27Glu*) 1.2 or 2.2 (C/G or G/G) or ADRB2 rs1042714 (27Gln/Gln) 1.1 (C/C), in combination with ADRB2 rs 1042713 (16Gly*) 1.2 or 1.1 (G/A or G/G) or ADRB3 rs4994 (64Arg*) 2.1 or 2.2 (C/T or C/C). These subjects absorb more of their dietary fat and tend to store it in fat cells, rather than mobilize it during metabolism. They tend to have impaired energy metabolism and to be less responsive to regular exercise for achieving their weight management goals. This composite genotype is expected in about 34 % of the Caucasian population.

Composite Genotype Pattern #6 - Responsive to Carbohydrate Restriction, Less Responsive to Exercise: Subjects whose genotypes include PPARG rs1801282 (12Ala/*) 1.2 or 2.2 (C/G or G/G) and/or ADRB2 rs1042714 (27Glu/*) 1.2 or 2.2 (C/G or G/G), as well as subjects with a combined genotype of PPARG rs1801282 (12Ala/*) 1.2 or 2.2 (C/G or G/G) and FABP2 rs1799883 (54Thr/*) 2.2 or 2.1 (A/A or A/G). All of the above qualifying genotypes will must also be in combination with ADRB2 rs1042713 (16Gly*) 1.2 or 1.1 (G/A or G/G) or ADRB3 rs4994 (64Arg*) 2.1 or 2.2 (C/T or C/C), to meet the less responsive to exercise requirement. These subjects tend to gain or retain weight from high dietary carbohydrate intake, and show signs of impaired glucose and insulin metabolism. They tend to have impaired energy metabolism and to be less responsive to regular exercise for achieving their weight management goals. This composite genotype is expected in about 40 % of the Caucasian population.

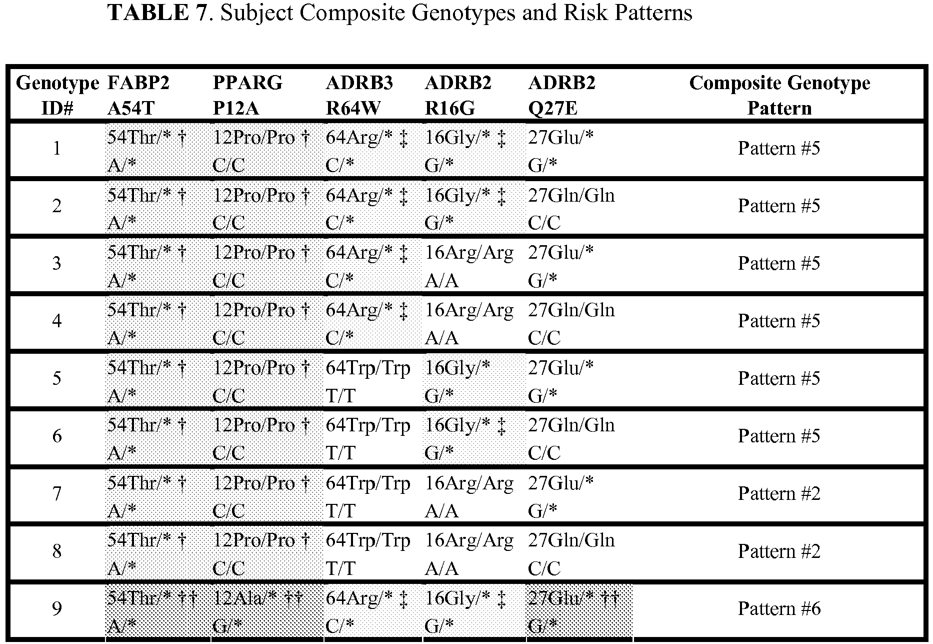

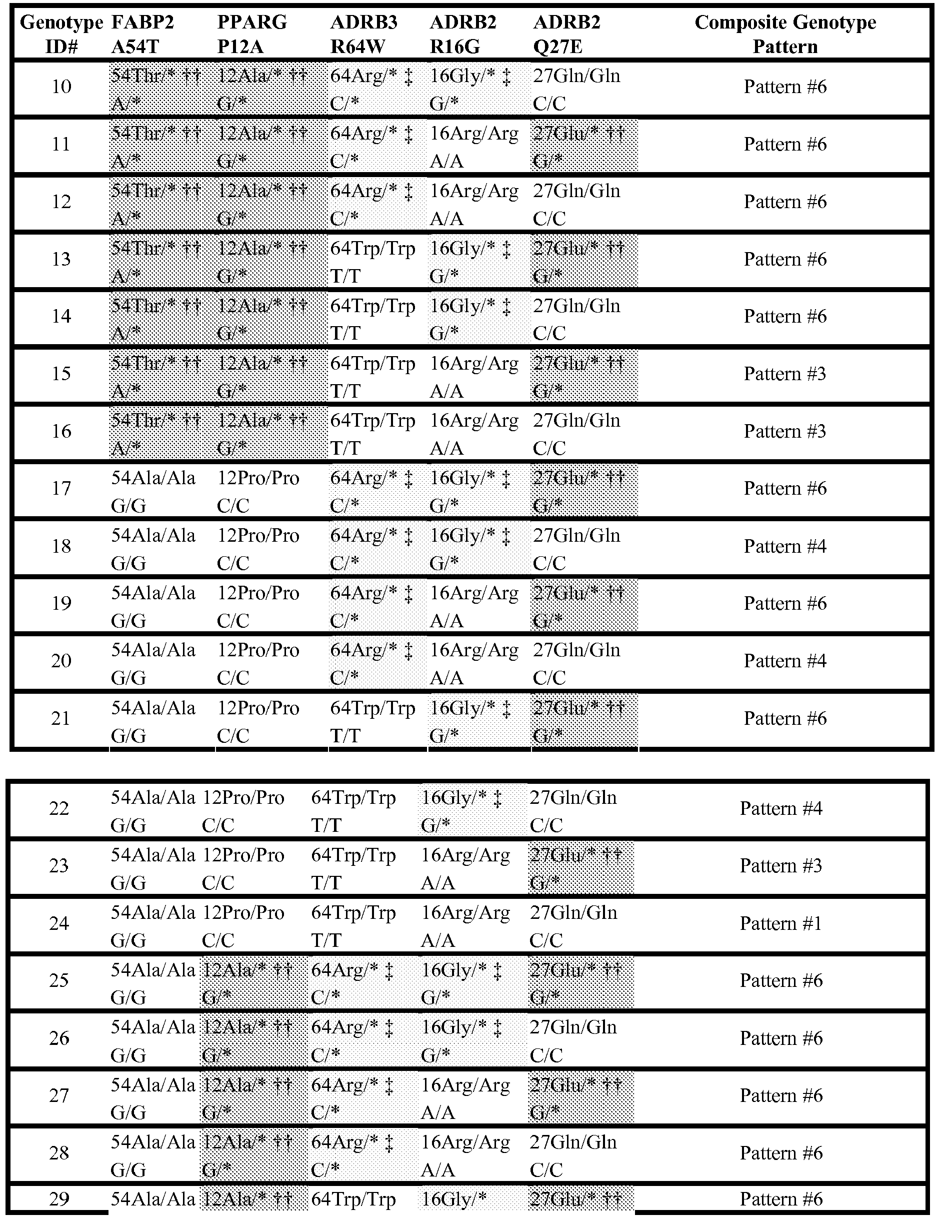

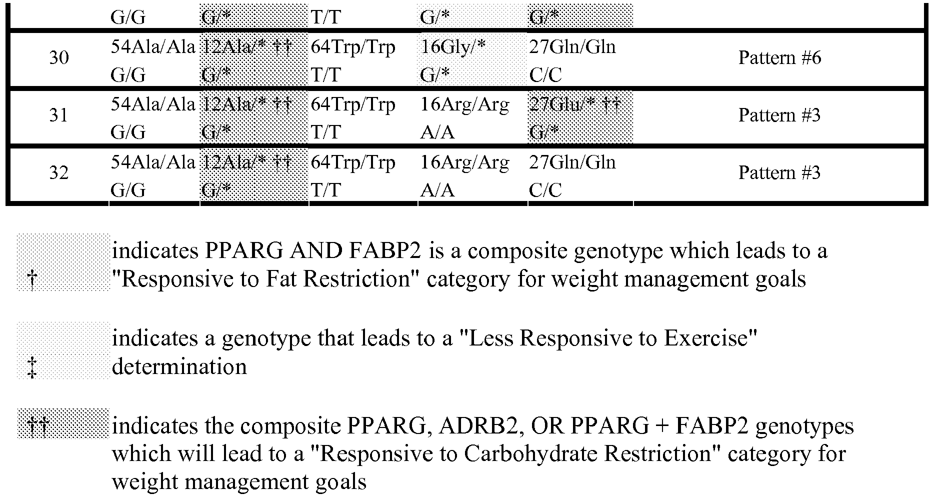

### EXAMPLE 2. CLINICAL GENOTYPING METHOD

DNA was either extracted from buccal swabs (SOP#12, version 1.3) or purchased from the Coriell Cell Repositories. The isolated DNA was used to PCR amplify regions of sequence surrounding five SNPs (SOP#29, version 1.0). The resulting four amplicons from each sample were treated with exonuclease I (Exo) and shrimp alkaline phosphatase (SAP) to remove excess primers and nucleotides (SOP#29, version 1.0). The purified amplicons were used in the single base extension (SBE) reaction with primers specific to its SNP target (SOP#30, version 1.0). Once the SBE was completed, SAP was again added to remove unincorporated nucleotides (SOP#30, version 1.0). The SBE product was then analyzed on the Beckman Coulter CEQ8800 with a standard of known migration size (SOP#15, version 1.4 and SOP#16, version 1.3). All genotypes, with the exception of PPARG (rs1801282), were assayed on the forward DNA strand. PPARG (rs1801282) was assayed on the reverse DNA strand and will be displayed as the complement base on the CEQ8800 traces. The resulting genotypes were recorded and then compared to the genotypes generated by DNA sequencing at Agencourt Bioscience Corporation or to known genotypes recorded at NCBI. Singleplex format: Subject PCR products were amplified separately and subjectly genotyped by the corresponding SBE primer. Poolplex format: Subject PCR products were amplified separately and then pooled together. The pooled DNA is genotyped for all five SNPs in a single reaction using a mixture of SBE primers. Multiplex format: All four PCR products were generated in a single reaction. The multiplexed PCR products were genotyped for all five SNPs in a single reaction using a mixture of SBE primers.

### Standardization

A commercially available size standard (Beckman Coulter part # 608395) was run with the samples as an internal reference for genotyping.

### Accuracy and Specificity

In order to insure that the correct genes were being targeted and accurately genotyped, the PCR products were submitted to an independent laboratory (Agencourt Bioscience Corporation) for sequencing and genotyping. At Agencourt, the sequence was compared to the genomic sequence flanking the SNP then the genotypes of each sample were reported to Interleukin Genetics. The Agencourt and Interleukin results were then compared for concordance.

### SNP Names And Abbreviations

The following SNP names and abbreviations are used in this assay validation: ADRB2 (R16G), rs1042713 = A1; ADRB2 (Q27E), rs1042714 = A2; ADRB3 (R64W), rs4994 = A3; FABP2 (A54T), rs1799883 = FA; PPARG (P12A), rs1801282=PP.

### Results

### PCR Results

Isolated DNA was PCR amplified using the primer sets listed in appendix B. ADRB2 (rs1042713) and ADRB2 (rs1042714) are 33 nucleotides apart and were amplified on a single PCR product. PCR products were run on agarose gels to verify the expected product sizes of: A1/A2 = 422 bp, A3 = 569 bp, FA = 311 bp, PP = 367 bp.

### Genotyping Results

### Peak Migration

Each SNP-specific single base extension primer was designed at a unique length to create a peak(s) at a specific location in relation to the known size standards when run on the CEQ8800 capillary electrophoresis instrument. The peak locations may not exactly match the primer sizes due to the effects of dye mobility, primer sequence and the analysis software, but they do migrate consistently. Single base extension primers are listed in Appendix C along with their expected peak migrations.

### Base calling

The single base extension reaction adds a fluorescently labeled base to the 3' end of the SNP-specific primer. This product is read by two lasers within the CEQ8800. The results are analyzed by the CEQ8800 software and appear as colored peaks- each color representing a different base. Presence of one single-colored peak at the specified locus indicates a homozygote while two peaks of different colors indicate a heterozygote. Within the thirty-nine samples that were genotyped in the validation are representatives of almost all homozygous and all heterozygous genotypes for all five SNPs. The one exception is a homozygous C genotype for the PPARG SNP. This was not unexpected since the frequency of the C allele in the general population is only 0.1 (as indicated by the dbSNP database for rs#1801282). However, the homozygous C genotype has been encountered in other samples outside the scope of this validation.

The CEQ8800 software features the ability for the user to specify SNP locus tags. The user indicates the migration size (in nucleotides) based on the expected migration of the SNP-specific primer. This enables the computer to identify a SNP based on its migration in relation to the standardized markers run along with the sample. The computer will also identify the base(s) within the SNP based on the dye indicator(s) it detects. For this validation, the computer was allowed to make the initial call of each SNP. The data was then independently re-analyzed by two technicians for confirmation. In all cases the computer calls and the two independent (manual) calls were in agreement.

### Coriell Samples

After genotyping had been performed in the singleplex format on the fifteen Coriell DNA samples, the results were compared to the known genotypes and were 100% concordant (see table 8).

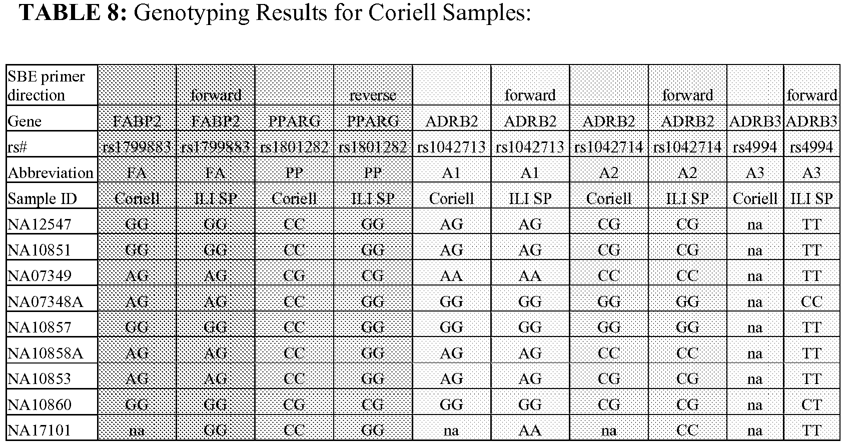

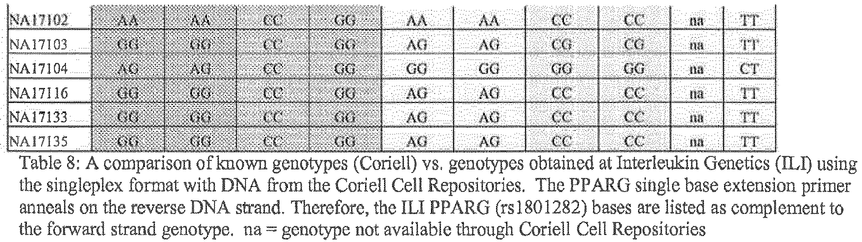

### REFERENCES

1. Baier LJ, Sacchettini JC, Knowler WC, Eads J, Paolisso G, Tataranni PA, Mochizuki H, Bennett PH, Bogardus C, and Prochazka M. An amino acid substitution in the human intestinal fatty acid binding protein is associated with increased fatty acid binding, increased fat oxidation, and insulin resistance. J Clin Invest 95: 1281-1287, 1995.
2. Levy E, Menard D, Delvin E, Stan S, Mitchell G, Lambert M, Ziv E, Feoli-Fonseca JC, and Seidman E. The polymorphism at codon 54 of the FABP2 gene increases fat absorption in human intestinal explants. J Biol Chem 276: 39679-39684, 2001.
3. Hegele RA, Harris SB, Hanley AJ, Sadikian S, Connelly PW, and Zinman B. Genetic variation of intestinal fatty acid-binding protein associated with variation in body mass in aboriginal Canadians. J Clin Endocrinol Metab 81: 4334-4337, 1996.
4. Yamada K, Yuan X, Ishiyama S, Koyama K, Ichikawa F, Koyanagi A, Koyama W, and Nonaka K. Association between Ala54Thr substitution of the fatty acid-binding protein 2 gene with insulin resistance and intra-abdominal fat thickness in Japanese men. Diabetologia 40: 706-710, 1997.
5. Albala C, Santos JL, Cifuentes M, Villarroel AC, Lera L, Liberman C, Angel B, and Perez-Bravo F. Intestinal FABP2 A54T polymorphism: association with insulin resistance and obesity in women. Obes Res 12: 340-345, 2004.
6. Pratley RE, Baier L, Pan DA, Salbe AD, Storlien L, Ravussin E, and Bogardus C. Effects of an Ala54Thr polymorphism in the intestinal fatty acid-binding protein on responses to dietary fat in humans. J Lipid Res 41: 2002-2008, 2000.
7. Agren JJ, Valve R, Vidgren H, Laakso M, and Uusitupa M. Postprandial lipemic response is modified by the polymorphism at codon 54 of the fatty acid-binding protein 2 gene. Arterioscler Thromb Vasc Biol 18: 1606-1610, 1998.
8. Agren JJ, Vidgren HM, Valve RS, Laakso M, and Uusitupa MI. Postprandial responses of subject fatty acids in subjects homozygous for the threonine- or alanine-encoding allele in codon 54 of the intestinal fatty acid binding protein 2 gene. Am J Clin Nutr 73: 31-35,2001.
9. Lefevre M, Lovejoy JC, Smith SR, Delany JP, Champagne C, Most MM, Denkins Y, de Jonge L, Rood J, and Bray GA. Comparison of the acute response to meals enriched with cis- or trans-fatty acids on glucose and lipids in overweight subjects with differing FABP2 genotypes. Metabolism 54: 1652-1658, 2005.
10. de Luis DA, Aller R, Izaola O, Gonzalez Sagrado M, and Conde R. Influence of ALA54THR Polymorphism of Fatty Acid Binding Protein 2 on Lifestyle Modification Response in Obese Subjects. Ann Nutr Metab 50: 354-360, 2006.
11. Marin C, Perez-Jimenz F, Gomez P, Delgado J, Paniagua A, Lozano A, Cortes B, Jimenez-Gomez Y, Gomez M, Lopez-Miranda J. The ala54 polymorphism of the fatty acid-binding protein 2 gene is associated with a change in insulin sensitivity after a change in the type of dietary fat. Am J Clin Nutr 82: 196-200, 2005.
12. Takakura Y, Yohsioka K, Umekawa T, Kogure A, Toda H, Yoshikawa T, Yoshida T. Thr54 allele of the FABP2 gene affects resting metabolic rate and visceral obesity. Diabetes Research and Clinical Practice 67: 36-42, 2005.
13. Jones JR, Barrick C, Kim K-A, Linder J, Blondeau B, et al, Deletion of PPARγ in adipose tissues of mice protects against high fat diet-induced obesity and insulin resistance. PNAS 102: 6207-6212, 2005.
14. Deeb SS, Fajas L, Nemoto M, Pihlajamaki J, Mykkanen L, Kuusisto J, Laakso M, Fujimoto W, and Auwerx J. A Pro12Ala substitution in PPARgamma2 associated with decreased receptor activity, lower body mass index and improved insulin sensitivity. Nat Genet 20: 284-287, 1998.
15. Rankinen T, Zuberi A, Chagnon YC, Weisnagel J, Argyropoulos G, et al. The human obesity gene map: The 2005 update. Obesity 14: 529-644.
16. Robitaille J, Despres JP, Perusse L, and Vohl MC. The PPAR-gamma P12A polymorphism modulates the relationship between dietary fat intake and components of the metabolic syndrome: results from the Quebec Family Study. Clin Genet 63: 109-116,2003.
17. Memisoglu A, Hu PJ, Hankinson SE, Manson JE, De Vivo I, Willet WC, and Hunter DJ. Interaction between a peroxisome proliferator-activated receptor gamma gene polymorphism and dietary fat intake in relation to body mass. Human Molecular Genetics 12: 2923-2929, 2001.
18. Lindi VI, Uusitupa MI, Lindstrom J, Louheranta A, Eriksson JG, Valle TT, Hamalainen H, Ilanne-Parikka P, Keinanen-Kiukaanniemi S, Laakso M, and Tuomilehto J. Association of the Pro12Ala polymorphism in the PPAR-gamma2 gene with 3-year incidence of type 2 diabetes and body weight change in the Finnish Diabetes Prevention Study. Diabetes 51: 2581-2586, 2002.
19. Nicklas BJ, van Rossum EF, Berman DM, Ryan AS, Dennis KE, and Shuldiner AR. Genetic variation in the peroxisome proliferator-activated receptor-gamma2 gene (Pro12Ala) affects metabolic responses to weight loss and subsequent weight regain. Diabetes 50: 2172-2176, 2001.
20. Meirhaeghe A, Helbecque N, Cottel D, Amouyel P. Impact of polymorphisms of the human β2-adrenoreceptor gene on obesity in a French population. Intntl J Obesity 24: 382-87, 2000.
21. Green SA, Turki J, Innis M, and Liggett SB. Amino-terminal polymorphisms of the human beta 2-adrenergic receptor impart distinct agonist-promoted regulatory properties. Biochemistry 33: 9414-9419, 1994.
22. Hellstrom L, Large V, Reynisdottir S, Wahrenberg H, Arner P. The different effects of a Gln27Glu B2-adrenoreceptor gene polymorphism on obesity in males and females. J Intern Med 245: 253-259, 1999.
23. Garenc C, Perusse L, Chagnon YC, Rankinen T, Gagnon J, Borecki IB, Leon AS, Skinner JS, Wilmore JH, Rao DC, and Bouchard C. Effects of beta2-adrenergic receptor gene variants on adiposity: the HERITAGE Family Study. Obes Res 11: 612-618,2003.
24. Lange LA, Norris JM, Langefeld CD, Nicklas BJ, Wagenknecht LE, Saad MF, and Bowden DW. Association of adipose tissue deposition and beta-2 adrenergic receptor variants: the IRAS family study. Int J Obes (Lond) 29: 449-457, 2005.
25. Gonzalez Sanchez JL, Proenza AM, Martinez Larrad MT, Ramis JM, Fernandez Perez C, Palou A, and Serrano Rios M. The glutamine 27 glutamic acid polymorphism of the beta2-adrenoceptor gene is associated with abdominal obesity and greater risk of impaired glucose tolerance in men but not in women: a population-based study in Spain. Clin Endocrinol (Oxf) 59: 476-481, 2003.
26. Masuo K, Katsuya T, Kawaguchi H, Fu Y, Rakuga H, et al. B2-adrenoreceptor polymorphisms relate to obesity through blunted leptin-mediated sympathetic activation. Am J Hypertens, 19:1084-91, 2006.
27. Ellsworth DL, Coady SA, Chen W, Srinivasan SR, Elkasabany A, Gustat J, Boerwinkle E, and Berenson GS. Influence of the beta2-adrenergic receptor Arg16Gly polymorphism on longitudinal changes in obesity from childhood through young adulthood in a biracial cohort: the Bogalusa Heart Study. Int J Obes Relat Metab Disord 26: 928-937, 2002.
28. Masuo K, Katsuya T, Fu Y, Rakugi H, Ogihara T, and Tuck ML. Beta2- and beta3-adrenergic receptor polymorphisms are related to the onset of weight gain and blood pressure elevation over 5 years. Circulation 111: 3429-3434, 2005.
29. van Rossum CT, Hoebee B, Seidell JC, Bouchard C, van Baak MA, de Groot CP, Chagnon M, de Graaf C, and Saris WH. Genetic factors as predictors of weight gain in young adult Dutch men and women. Int J Obes Relat Metab Disord 26: 517-528, 2002.
30. Martinez JA, Corbalan MS, Sanchez-Villegas A, Forga L, Marti A, and Martinez-Gonzalez MA. Obesity risk is associated with carbohydrate intake in women carrying the Gln27Glu beta2-adrenoceptor polymorphism. J Nutr 133: 2549-2554, 2003.
31. Ukkola O, Tremblay A, and Bouchard C. Beta-2 adrenergic receptor variants are associated with subcutaneous fat accumulation in response to long-term overfeeding. Int J Obes Relat Metab Disord 25: 1604-1608, 2001.
32. Corbalan MS. The 27Glu polymorphism of the beta2-adrenergic receptor gene interacts with physical activity influencing obesity risk among female subjects. Clin Genet 61: 305-307, 2002.
33. Umekawa T, Yoshida T, Sakane N, Kogure A, Kondo M, and Honjyo H. Arg64Trp mutation of beta3-adrenoceptor gene deteriorates lipolysis induced by beta3-adrenoceptor agonist in human omental adipocytes. Diabetes 48: 117-120, 1999.
34. Hoffstedt J, Poirier O, Thorne A, Lonnqvist F, Herrmann SM, Cambien F, and Arner P. Polymorphism of the human beta3-adrenoceptor gene forms a well-conserved haplotype that is associated with moderate obesity and altered receptor function. Diabetes 48: 203-205, 1999.
35. Allison DB, Heo M, Faith MS, and Pietrobelli A. Meta-analysis of the association of the Arg64Trp polymorphism in the beta3 adrenergic receptor with body mass index. Int J Obes Relat Metab Disord 22: 559-566, 1998.
36. Fujisawa T, Ikegami H, Kawaguchi Y, and Ogihara T. Meta-analysis of the association of Arg64Trp polymorphism of beta 3-adrenergic receptor gene with body mass index. J Clin Endocrinol Metab 83: 2441-2444, 1998.
37. Kurokawa N, Nakai K, Kameo S, Liu ZM, and Satoh H. Association of BMI with the beta3-adrenergic receptor gene polymorphism in Japanese: meta-analysis. Obes Res 9: 741-745,2001.
38. Marti A, Corbalan MS, Martinez-Gonzalez MA, and Martinez JA. ARG64TRP polymorphism of the beta 3-adrenergic receptor gene and obesity risk: effect modification by a sedentary lifestyle. Diabetes Obes Metab 4: 428-430, 2002.
39. Sakane N, Yoshida T, Umekawa T, Kogure A, Takakura Y, and Kondo M. Effects of Arg64Trp mutation in the beta 3-adrenergic receptor gene on weight loss, body fat distribution, glycemic control, and insulin resistance in obese type 2 diabetic patients. Diabetes Care 20: 1887-1890, 1997.
40. Shiwaku K, Nogi A, Anuurad E, Kitajima K, Enkhmaa B, Shimono K, and Yamane Y. Difficulty in losing weight by behavioral intervention for women with Arg64Trp polymorphism of the beta3-adrenergic receptor gene. Int J Obes Relat Metab Disord 27: 1028-1036, 2003.
41. Phares DA, Halverstadt AA, Shuldiner AR, Ferrell RE, Douglass LW, Ryan AS, Goldberg AP, and Hagberg JM. Association between body fat response to exercise training and multilocus ADR genotypes. Obes Res 12: 807-815, 2004.
42. Tchernof A, Starling RD, Walston JD, Shuldiner AR, et al. Obesity-related phenotypes and the β3-adrenoreceptor gene variant in postmenopausal women. Diabetes 48:1425-1428, 1999.
43. Deeb SS, Fajas L, Nemoto M, Pihlajamaki J, Mykkanen L, Kuusisto J, Laakso M, Fujimoto W, and Auwerx J. A Pro12Ala substitution in PPARgamma2 associated with decreased receptor activity, lower body mass index and improved insulin sensitivity. Nat Genet 20: 284-287, 1998.
44. Masugi J, Tamori Y, Mori H, Koike T, and Kasuga M. Inhibitory effect of a proline-to-alanine substitution at codon 12 of peroxisome proliferator-activated receptor-gamma 2 on thiazolidinedione-induced adipogenesis. Biochem Biophys Res Commun 268: 178-182, 2000.
45. Kahara T, Takamura T, Hayakawa T, Nagai Y, Yamaguchi H, Katsuki T, Katsuki K, Katsuki M, and Kobayashi K. PPARgamma gene polymorphism is associated with exercise-mediated changes of insulin resistance in healthy men. Metabolism 52: 209-212,2003.
46. Adamo KB, Sigal RJ, Williams K, Kenny G, Prud'homme D, and Tesson F. Influence of Pro12Ala peroxisome proliferator-activated receptor gamma2 polymorphism on glucose response to exercise training in type 2 diabetes. Diabetologia 48: 1503-1509, 2005.
47. Weiss EP, Kulaputana O, Ghiu IA, Brandauer J, Wohn CR, Phares DA, Shuldiner AR, and Hagberg JM. Endurance training-induced changes in the insulin response to oral glucose are associated with the peroxisome proliferator-activated receptor-gamma2 Pro12Ala genotype in men but not in women. Metabolism 54: 97-102, 2005.
48. Guettier J, Georgopoulos A, Tsai M, Radha V, Shanthrani S, Deepa R, Gross M, Rao G, Mohan V. Polymorphisms in the fatty acid-binding protein 2 and apolipoprotein c-III genes are associated with the metabolic syndrome and dyslipidemia in a south Indian population. J Clin Endocrinol Metab 90: 1705-1711, 2004.
49. Pollex R, Hanley A, Zinman B, Harris S, Khan H, Hegele R. Metabolic syndrome in aboriginal Canadians: prevalence and genetic associations. Atherosclerosis 184:121-129,2006.
50. Karani S, Radha V, Mohan V. Thr54 allele carriers of the Ala54Thr variant of FABP2 gene have associations with metabolic syndrome and hypertriglyceridemia in urban South Indians. Metabolism Clinical and Experimental 55: 1222-12226, 2006.
51. Pereira M, Swain J, Goldfine A, Rifai N, Ludwig D. Effects of a low-glycemic load diet on resting energy expenditure and heart disease risk factors during weight loss. JAMA 292(20): 2482-2490, 2004.
52. Hallikainen M, Toppinen L, Mykkanen H, Agren J, Laaksonen D, Miettinen T, Niskanen L, Poutanen K, Gylling H. Interaction between cholesterol and glucose metabolism during dietary carbohydrate modification in subjects with the metabolic syndrome. Am J Clin Nutr 84: 1385-1392, 2006.
53. Kallio P, Kolehmainen M, Laaksonen D, Kekalainen J, Salopuro T, Sivenius K, Pulkkinen L, Mykkanen H, Niskanen L, Uusitupa M, Poutanen K. Dietary carbohydrate modification induces alterations in gene expression in abdominal subcutaneous adipose tissue in persons with the metabolic syndrome: the FUNGENUT study. Am J Clin Nutr 85: 1417-1427, 2007.
54. Paradis A-M, Fontaine-Bisson B, Bosse Y, Robitaille J, Lemieux S, Jaques H, Lamarche B, Tchernof A, Couture P, Vohl M-C, The peroxisome proliferator-activated receptor α Leu162Val polymorphism influences the metabolic response to a dietary intervention altering fatty acid proportions in healthy men. Am J Clin Nutr 81: 523-30, 2005.
55. Macho-Azcarate T, Marti A, Gonzalez A, Martinez JA, Ibanez J. Gln27Glu polymorphism in the beta2 adrenergic receptor gene and lipid metabolism during exercise in obese women. Int J Obesity 26: 1434-41, 2002.
56. Kahara T, Hayakawa T, Nagai Y, Shimizu A, Takamura T. Gln27Glu polymorphism of the β2 adrenergic receptor gene in healthy Japanese men is associated with the change of fructosamine level caused by exercise. Diabet Res Clin Practice 64: 207-12, 2004.
57. Marti A, Corbalan MS, Martinez-Gonzalez MA. CHO intake alters obesity risk associated with Pro12Ala polymorphism of PPARG gene. J. Physiol. Biochem., 58(4): 219-220,2002.
58. Centers for Disease Control and Prevention, available at http://www.cdc.gov/nccdphp/dnpa/obesitv/trend/maps/index.htm. Accessed 10/21/07.
59. National Center for Health Statistics, available at http://www.cdc.gov/nchs/fastat/overwt.htm. Accessed 10/21/07.
60. Flegal KM, Carroll MD, Ogden CL, Johnson CL. Prevalence and trends in obesity among US adults, 1999-2000. JAMA, 288:1723-1727, 2002.
61. Ogden CL, Carroll MD, Curtin LR, McDowell MA, Tabak CJ, Flegal KM. Prevalence of overweight and obesity in the United States, 1999-2004. JAMA, 295:1549-155,2006.
62. Ogden CL, Flegal KM, Carroll MD, Johnson CL. Prevalence and trends in overweight among US children and adolescents, 1999-2000. JAMA 288:1728-1732, 2002.
63. Centers for Disease Control and Prevention, available at http://www.cdc.gov/nccdphp/dnpa/obesity/consequences.htm. Accessed 10/21/07.
64. Centers for Disease Control and Prevention, available at http://www.cdc.gov/nccdph/dnpa/obesity/economic_consequences.htm. Accessed 10/21/07.
65. Wolf AM, Colditz GA. Current estimates of the economic cost of obesity in the United States. Obes Res 6:97-106, 1998.
66. Finkelstein, EA, Fiebelkorn, IC, Wang, G. National medical spending attributable to overweight and obesity: How much, and who's paying? Health Affairs Suppl. W3; 219-226, 2003.
67. U.S. Department of Health and Human Services. The Surgeon General's Call to Action to Prevent and Decrease Overweight and Obesity. Rockville, MD: U.S. Department of Health and Human Services, Public Health Service, Office of the Surgeon General; 2001.
68. Johnson R, Williams S, Spruill I. Genomics, nutrition, obesity and diabetes. J Nurs Scholarsh 38:11-18, 2006.
69. Frosch D, Mello P, Lerman C. Behavioral consequences of testing for obesity risk. Cancer Epidemiol Biomarkers Prev 14:1485-1489, 2005.
70. World Health Organization. BMI Classification.

## Claims

1. A method for selecting an appropriate therapeutic/dietary regimen recommendation for a subject comprising determining the subject's genotype with respect to the FABP2 (rs1799883; G/A); PPARG (rs1801282; C/G); and ADRB2 (rs1042714; C/G) polymorphic loci and at least one of the ADRB3 (rs4994; C/T) locus and the ADRB2 (rs1042713; A/G) locus, wherein the subject's genotype with respect to said loci provides information about the subject's increased susceptibility to adverse weight management issues, and allows the selection of a therapeutic/dietary regimen or lifestyle recommendation that is suitable to the subject's susceptibility to adverse weight management issues and wherein carrying ADRB2 Glu27 allele, PPARG 12Pro/Pro and FABP2 54Thr/* qualifies the subject as responsive to fat restriction.

2. The method of claim 1, which comprises determining the subject's genotype with respect to both the ADRB3 (rs4994 C/T) locus and the ADRB2 (rs1042713 A/G) locus.

3. The method according claim 1, wherein the therapeutic/dietary regimen comprises administering a nutraceutical.

## Patentansprüche

1. Verfahren zum Wählen einer geeigneten Therapie-/Diätplanempfehlung für ein Individuum, umfassend: Bestimmen des Genotyps des Individuums bezüglich der polymorphen Loci FABP2 (rs1799883; G/A); PPARG (rs1801282; C/G); und ADRB2 (rs1042714; C/G) und wenigstens eines der Loci ADRB3 (rs4994; C/T) und ADRB2 (rs1042713; A/G), wobei der Genotyp des Individuums bezüglich dieser Loci Informationen über die erhöhte Anfälligkeit des Individuums gegenüber unerwünschten Problemen bei der Gewichtskontrolle liefert und die Wahl einer Empfehlung für einen Therapie-/Diätplan oder für eine Lebensweise erlaubt, die für die Anfälligkeit des Individuums gegenüber unerwünschten Problemen bei der Gewichtskontrolle geeignet ist, und wobei Tragen des ADRB2 Glu27-Allels, von PPARG 12Pro/Pro und von FABP2 54Thr/* das Individuum dafür qualifiziert, auf Fettrestriktion zu reagieren.

2. Verfahren nach Anspruch 1, welches Bestimmen des Genotyps des Individuums bezüglich sowohl des ADRB3 (rs4994 C/T)-Lokus als auch des ADRB2 (rs1042713 A/G)-Lokus umfasst.

3. Verfahren nach Anspruch 1, wobei der Therapie-/Diätplan die Verabreichung eines Nutrazeutikums umfasst.

## Revendications

1. Procédé pour sélectionner une recommandation de régime thérapeutique/alimentaire approprié pour un sujet, comprenant la détermination du génotype du sujet en ce qui concerne les loci polymorphes FABP2 (rs1799883 ; G/A) ; PPARG (rs1801282 ; C/G) ; et ADRB2 (rs1042714 ; C/G) et au moins l'un parmi le locus ADRB3 (rs4494 ; C/T) et le locus ADRB2 (rs1042713 ; A/G), dans lequel le génotype du sujet en ce qui concerne lesdits loci donne des informations concernant la plus grande susceptibilité du sujet à présenter des problèmes indésirables de gestion du poids, et permet la sélection d'une recommandation de régime thérapeutique/alimentaire ou de style de vie adapté à la susceptibilité du sujet à présenter des problèmes indésirables de gestion du poids, et dans lequel le fait de porter l'allèle ADRB2 Glu27, PPARG 12Pro/Pro et FABP2 54THR/* qualifie le sujet comme étant sensible à une limitation des graisses.

2. Procédé selon la revendication 1, qui comprend la détermination du génotype du sujet en ce qui concerne tant le locus ADRB3 (rs4994 C/T) que le locus ADRB2 (rs1042713 A/G).

3. Procédé selon la revendication 1, dans lequel le régime thérapeutique/alimentaire comprend l'administration d'un nutraceutique.
